# EUROPÄISCHE PATENTSCHRIFT

(11) **EP 3 665 551 B1**
(45) Veröffentlichungstag und Bekanntmachung des Hinweises auf die Patenterteilung: **23.07.2025**
(21) Anmeldenummer: 18792863.5
(22) Anmeldetag: 10.10.2018
(51) Int. Cl.: G06F 3/01, G06F 3/04815, G06F 3/04817, G06F 3/04842, G06F 3/0488, G06F 3/0486, G06F 3/04847, G06F 3/00, G06F 3/03, G06F 30/00, G16H 40/20

(54) **VERFAHREN ZUM VIRTUELLEN KONFIGURIEREN EINER EINRICHTUNG, COMPUTERPROGRAMMPRODUKT UND ENTSPRECHENDES AUGMENTED REALITY SYSTEM**
METHOD FOR VIRTUALLY CONFIGURING AN INSTALLATION, COMPUTER PROGRAM PRODUCT AND CORRESPONDING AUGMENTED REALITY SYSTEM
PROCÉDÉ DE CONFIGURATION VIRTUELLE D'UNE INSTALLATION, PRODUIT-PROGRAMME D'ORDINATEUR ET SYSTÈME CORRESPONDANT DE RÉALITÉ AUGMENTÉE

(30) Priorität: 30.10.2017 DE 102017010190
(43) Veröffentlichungstag der Anmeldung: 17.06.2020
(73) Patentinhaber: Sartorius Stedim Biotech GmbH, 37079 Göttingen (DE)
(72) Erfinder: SCHMELIG, Christian, 34327 Körle (DE); SCHLACK, Stefan, 37083 Göttingen (DE)
(74) Vertreter: Novagraaf International SA
(86) Internationale Anmeldenummer: PCT/EP2018/077534
(87) Internationale Veröffentlichungsnummer: WO 2019/086214

(56) Entgegenhaltungen:
- EP-A1- 3 165 979
- WO-A2-2015/075705
- US-A1- 2007 156 540
- US-A1- 2017 076 500

## Beschreibung

Die vorliegende Erfindung betrifft ein Verfahren zum virtuellen Konfigurieren einer Einrichtung, ein entsprechendes Computerprogrammprodukt sowie ein entsprechendes Augmented Reality System. Insbesondere ist ein verbessertes Verfahren zur Konfiguration und/oder Anpassung und/oder Individualisierung und/oder Optimierung einer Einrichtung, insbesondere einer Bioprozesseinrichtung bereitgestellt. Die Erfindung kann insbesondere in den folgenden Bereichen Anwendung finden: Biotechnologie, biopharmazeutische und pharmazeutische Industrie, Medizintechnik, chemische Industrie, physikalische Technik, Lebensmitteltechnologie, Prozessierungstechnologie und ähnliche. Besonders bevorzugt ist die interaktive Verwendung des Verfahrens durch einen Verwender, insbesondere einen Kunden, der eine Bestellung über eine selbstkonfigurierte Einrichtung bei einem Anbieter/Verkäufer beispielsweise online platzieren möchte.

Bisher ist es üblich, dass eine Einrichtung konfiguriert wird, indem reale Einrichtungselemente in einer realen Umgebung positioniert und/oder aufgebaut und/oder zusammengesetzt und/oder ausprobiert und/oder in Betrieb genommen werden. Zu diesem Zweck werden der Betriebszustand und/oder die Funktionalität und insbesondere die korrekte Verbindung der zu montierenden realen Einrichtungselemente visuell und/oder manuell überprüft. Vor der Inbetriebnahme der Einrichtung muss dementsprechend ein Verwender alle Kompatibilitäten und/oder Anschlüsse überprüfen, insbesondere unter Einbeziehung technischer Datenblätter und der Auswahl geeigneter und kompatibler realer Einrichtungselemente.

Mit wachsender Komplexität von Anlagen und Einrichtungen werden effektivere und insbesondere effizientere Verfahren zur Konfiguration und/oder Optimierung und/oder für Testläufe benötigt. Insbesondere die biopharmazeutische Industrie, die strengen Vorschriften und Aufzeichnungsanforderungen unterliegt, ist auf einen weitestgehend verlässlichen und störungsfreien Betrieb von Anlagen und Einrichtungen angewiesen.

Darüber hinaus erfordern die meist sehr hochwertigen Produkte, welche innerhalb einer Einrichtung produziert und/oder verwendet werden, beispielsweise in single use Behältern eines Bioreaktors, einwandfrei funktionierende Einrichtungselemente und/oder das einwandfreie Zusammenspiel mehrerer Einrichtungselemente. Dies setzt voraus, dass bereits im Zuge der Konfiguration einer Einrichtung eine fehlerhafte Montage vermieden wird.

US 2017/076500 A1 beschreibt ein Verfahren für einen Mess- oder Produktionsaufbau welcher das Bereitstellen eines Visualisierungssystems mit einem Prozessor, einem Ausgabegerät und einem Erfassungsmodul zum Erfassen von Identifikationsdaten eines Arbeitsraums umfasst. Das Verfahren stellt ferner eine erste Einrichtungskomponente bereit, die mindestens eine erste Markierung im Arbeitsraum aufweist, und stellt eine zweite Einrichtungskomponente bereit, die mindestens eine zweite Markierung im Arbeitsraum aufweist und über mindestens eine Verbindung mit der ersten Einrichtungskomponente verbunden werden kann. Das Verfahren erfasst Identifikationsdaten über die Marker und identifiziert die Marker. Der Prozessor ruft digitale Informationen für die Marker aus einer Datenbank ab und trifft eine Entscheidung über die Verbindung der Setup-Komponenten basierend auf den abgerufenen digitalen Informationen und den erfassten Identifikationsdaten. Das Verfahren gibt dann eine visuelle Darstellung der Verbindung zwischen der ersten und der zweiten Einrichtungskomponente gemäß der Entscheidung aus. Das Visualisierungssystem kann ein Augmented-Reality System sein.

Der Erfindung liegt daher die Aufgabe zugrunde, ein Verfahren, ein Computerprogrammprodukt und ein Augmented Reality-System bereitzustellen, die jeweils eine effiziente Konfiguration einer Einrichtung ermöglichen.

Die Aufgabe wird durch die unabhängigen Ansprüche gelöst. Besonders bevorzugte Ausführungsformen sind Gegenstand der abhängigen Ansprüche.

Die Erfindung betrifft ein Verfahren zum virtuellen Konfigurieren einer Einrichtung, insbesondere einer Bioprozesseinrichtung, gemäß dem Anspruch 1.

Unter einer Bioprozesseinrichtung werden Gegenstände/Elemente insbesondere für die Prozessierung biologischer Medien, sowie deren systemische Kombination, beispielsweise in modularer Form, in Systemen verstanden. Ein solches System und/oder ein solches Element kann z.B. ein Bioreaktor, ein Einwegbeutel, ein Behältnis, ein Tank, ein Filtersystem, ein Mischtank, ein Gärtank etc. sein bzw. umfassen, welche als einzelne Gegenstände oder in sogenannten Prozesseinheiten (Unit Operations) angeordnet sein können. Diese können in Räumlichkeiten untergebracht sein, wie z.B. in Laboren, Reinräumen, Herstellungs- und Produktionshallen, in denen z.B. biopharmazeutische Wirkstoffe und/oder functional food entwickelt und hergestellt werden. Die Erfindung kann insbesondere in der pharmazeutischen und/oder biopharmazeutischen Industrie und/oder Lebensmittel- und/oder Getränkeindustrie Anwendung finden.

Ein Medium kann insbesondere ein Gas und/oder eine Flüssigkeit und/oder ein Feststoff, beispielsweise ein Granulat sein. Insbesondere kann/können mit dem Begriff "Medium" eine Dispersion, insbesondere eine Emulsion, eine Suspension, ein Gel und/oder ein Schaum gemeint sein. Besonders bevorzugt umfasst ein Medium einen biologischen und/oder einen chemischen Stoff, beispielsweise Zellen, Pflanzen, Bakterien und/oder deren Bestandteile und/oder Produkte. Beispielsweise kann es sich bei einem Medium um ein Zellkulturmedium und/oder ein Puffersystem handeln. Ganz besonders bevorzugt ist das Medium ein Fluid bzw. hat das Medium fluide Eigenschaften. Ein Medium kann ferner auch einen Stoff bzw. Stoffgemisch umfassen, der zur Herstellung und/oder als Bestandteil eines Lebensmittels, insbesondere eines sogenannten funktionalen Lebensmittels und/oder "functional food" dient. Ein Medium kann in anderen Worten insbesondere jeder Stoff bzw. alles sein, was fließen kann.

Insbesondere wird, basierend auf einem Augmented Reality-System bzw. einem System zur Erzeugung einer erweiterten Realität, welches einen Rechner bzw. eine Recheneinheit und mindestens eine Sensor- bzw. Detektoreinheit umfasst, folgendes Verfahren ausgeführt:
- Aufnehmen von Sensordaten mittels der Sensoreinheit bzw. Einträgen eines Sensordatensatzes, welche einer physischen Umgebung entsprechen bzw. eine physische Umgebung zumindest teilweise charakterisieren;
- Übersetzen bzw. Transformieren bzw. Digitalisieren der physischen Umgebung in eine virtuelle Umgebung basierend auf Einträgen des Sensordatensatzes, insbesondere umfassend ein Rekonstruieren und/oder Abbilden der physischen Umgebung in einem virtuellen Format;
- Virtuelles Anordnen bzw. Positionieren eines virtuellen Einrichtungselements, dem Einträge eines Parameterdatensatzes zugeordnet sind, innerhalb und/oder bezüglich der virtuellen Umgebung;
- Virtuelles Ausprobieren, insbesondere ein Abgleich von Kompatibilitäten und/oder ein Simulieren der Einrichtung und deren Funktion basierend auf den Einträgen des Parameterdatensatzes und des Sensordatensatzes.

Somit wird vorteilhaft ein Verfahren ermöglicht, welches eine reale physische Umgebung und bevorzugt ein physisches Einrichtungselement in eine virtuelle Darstellung bzw. eine virtuelle Umgebung übersetzt und/oder digitalisiert. In anderen Worten werden physische Aspekte einer realen Umgebung digitalisiert bzw. digital erfasst. Dabei können virtuelle Einrichtungselemente der virtuellen Umgebung virtuell hinzugefügt werden. Insbesondere ist ein virtuelles Einrichtungselement im Wesentlichen in seinen virtuellen Eigenschaften ein Modell, mindestens ein näherungsweises Modell eines realen physischen Einrichtungselements bzw. entspricht in seinen virtuellen Eigenschaften zumindest teilweise den Eigenschaften eines realen physischen Einrichtungselements.

Die virtuellen Eigenschaften eines virtuellen Einrichtungselements werden durch Kenndaten, die typischerweise unter anderem aus entsprechenden Datenblättern und/oder einer Eigenschaftsdatenbank entnehmbar sind, festgelegt. Solche Kenndaten können Bestandteil eines Parameterdatensatzes sein bzw. umfassen. Dies entspricht ebenfalls einer Art der Digitalisierung und/oder Modellierung eines physischen Einrichtungselements. Folglich entspricht eine virtuelle Umgebung, die virtuelle Einrichtungselemente umfasst, einem digitalen Modell einer realen physischen Umgebung, die ein oder mehrere physische Einrichtungselemente umfasst. Das Modell kann dabei beliebig detailgenau sein, also durch beliebig viele Parameter, welche unter anderem durch die physische Umgebung vorgegeben sind, gekennzeichnet sein.

Es ist somit dem Verwender vorteilhaft ermöglicht, eine virtuelle Einrichtung, insbesondere eine Bioprozesseinrichtung oder beispielsweise einen Laboraufbau für biotechnologische Anwendungen besonders effizient und intuitiv und insbesondere interaktiv, Computer-gestützt zu konfigurieren. Die virtuelle Einrichtung umfasst dabei mindestens ein virtuelles Einrichtungselement. Die Konfiguration findet im Wesentlichen statt bevor das physische Einrichtungselement, welches dem mindestens einen virtuellen Einrichtungselement entspricht, installiert wird, wobei es hierfür häufig bestellt und tatsächlich angeschafft werden muss.

Auf die besagte Weise kann ein Anwender des Verfahrens, der daran interessiert ist, ein oder mehrere entsprechende Einrichtungselemente zu installieren, dieses vorab ausprobieren bzw. testen, ob es seinen Anforderungen entspricht und/oder ob es überhaupt mit anderen, insbesondere bereits vorhandenen physischen Einrichtungselementen kompatibel ist. Bevorzugt kann der Verwender prüfen und/oder testen und/oder ausprobieren, ob die Einrichtung im Wesentlichen fehlerfrei betrieben werden kann.

Das beschriebene Verfahren ist insbesondere vorteilhaft für die Konfiguration komplexer Geräte und Anlagen bzw. Einrichtungen, die durch eine Vielzahl von Parametern eines Parameterdatensatzes definiert sind. Es ist demnach nicht notwendig, ein besagtes Gerät oder eine Anlage bzw. Einrichtung tatsächlich zu installieren, um diese in der physischen Umgebung zu testen bzw. auszuprobieren. Das Testen bzw. Ausprobieren des Gerätes und/oder der Einrichtung findet virtuell in einer erweiterten Realität, auch Augmented Reality genannt, in beliebig komplexer Ausführung und unter Einbeziehung beliebig vieler Parameter statt und sagt insbesondere voraus, ob die korrekte fehlerfreie Funktion gegeben ist oder nicht. In anderen Worten kann virtuell bereits herausgefunden oder vorhergesagt werden, ob eine Einrichtung, wie sie beispielsweise von einem Verwender gewünscht oder zunächst konfiguriert wird, fehlerfrei aufgebaut und/oder angeschlossen und/oder eingerichtet und/oder betrieben werden kann. Dabei ist es besonders bevorzugt, eine Funktion bzw. Funktionalität und/oder einen Betriebszustand simulieren zu können, sodass der Verwender auch eine Information darüber erhält, ob die gewünschte Konfiguration die für seine Zwecke und Ansprüche geeignete Funktionalität aufweist.

Eine Funktion kann beispielsweise Prozessschritte, welche durch ein Element oder die gesamte Einrichtung ausgeführt werden, umfassen und insbesondere eine Temperierung, ein Vermischen des Mediums, eine Zugabe eines Stoffes zu dem Medium, ein Druck-Auf- oder -Abbau, ein Ablassen eines Teils des Mediums und/oder andere Schritte umfassen. Ein Betriebszustand kann die Kompatibilität bzw. das "Zueinanderpassen" von Elementen, das "Hineinpassen" der Elemente in den Raum und/oder einen konkreten Zustand, wie beispielsweise "Behälter leer" oder "Behälter gefüllt", "Ventil offen", "Ventil geschlossen" "Element auf Betriebstemperatur" oder ähnliches umfassen. Es kann auch sein, dass sich eine "Funktion und/oder Funktionalität" nicht klar von einem "Betriebszustand" unterscheidet, weshalb unter Umständen mit den Begrifflichkeiten "Funktion und/oder Funktionalität" und "Betriebszustand" dasselbe gemeint sein kann.

Der Verwender und/oder der Vertreiber physischer Einrichtungselemente kann/können mittels des beschriebenen Verfahrens somit einen erheblichen Kosten- und Zeitaufwand minimieren, indem eine Konfiguration einer komplexen Einrichtung und/oder Anlage vorab virtuell interaktiv und Computer-gestützt stattfindet bzw. entsprechende Konfigurationsfehler vermeiden. Es kann insbesondere vermieden werden, dass physische Einrichtungselemente, wie beispielsweise biotechnologische Anlagen und/oder Bioreaktoren "auf Verdacht" installiert (insbesondere hergestellt und zusammengebaut, angeschafft und/oder angeliefert) werden, wobei die Gefahr bestehen kann, dass eine fehlerhafte Funktion und/oder Kompatibilität insbesondere in einer Wechselwirkung mit der physischen Umgebung und/oder anderen physischen Einrichtungselementen dann vor Ort nach Installation festgestellt wird. In dem Fall müsste das unpassende physische Einrichtungselement, welches angeschafft oder mindestens angeliefert wurde, wieder zurückgeliefert werden, was einen unerwünschten Zeit- und Kostenaufwand für den Verwender und/oder den Anbieter verursachen würde.

Überdies kann vorteilhaft vermieden werden, dass ein Kunde und/oder ein Verwender möglicherweise eine Mehrzahl komplizierter Datenblätter und/oder Kataloge studieren und/oder Datenbanken abfragen muss, um ein passendes und kompatibles Einrichtungselement zu ermitteln und auszuwählen. Das beschriebene Verfahren hat den Vorteil, eine besonders intuitive, effiziente und anwendungsfreundliche Möglichkeit zur Konfiguration einer Einrichtung bereitzustellen.

Die zumindest eine Sensoreinheit umfasst eine Kamera bzw. einen Fotoapparat. Die Kamera kann insbesondere eine 3D-fähige Kamera sein, also eine Kamera, die in der Lage ist, zwei- bzw. dreidimensionale Koordinaten eines Raumes bzw. einer Umgebung zu erfassen. Der Schritt des Detektierens des der physischen Umgebung zugehörigen Sensordatensatzes umfasst einen Schritt eines Aufnehmens bzw. Erfassens eines Bildes der physischen Umgebung. Der Sensordatensatz umfasst eine räumliche Information, insbesondere eine 3D-Topographie und/oder dreidimensionale (3D-)Koordinaten eines Raumes, und eine Gravitationsrichtung. Es wird ein Foto von einer physischen realen Umgebung, beispielsweise eines Labors aufgenommen. Bevorzugt umfasst das Foto Daten, die dreidimensionale Koordinaten enthalten oder aus denen dreidimensionale Koordinaten abgeleitet werden können. Solche Daten entsprechen beispielsweise Einträgen eines Sensordatensatzes.

Die Erfassung räumlicher Eigenschaften einer physischen bzw. realen Umgebung erlaubt, dass eine virtuelle 3D-Umgebung als Abbild der physischen Umgebung rekonstruiert werden kann. Dies ist insbesondere von Vorteil zur Ermittlung von Abständen und zum Abgleich von Dimensionen. Beispielsweise kann ermittelt werden, ob ein Einrichtungselement ausreichend Platz an dem gewünschten Bestimmungsort hat. Auch können physische Komponenten, beispielsweise Teile einer Laboreinrichtung und/oder Möbelstücke insbesondere als Abstellflächen erkannt werden und für die virtuelle Positionierung bzw. Anordnung von virtuellen Einrichtungselementen in Betracht kommen.

Eine Gravitationsrichtung, welche beispielsweise durch eine Markierung im Raum, hier Gravitationsmarkierung genannt, gegeben sein kann, wird erfasst. Die Ermittlung der Gravitationsrichtung erfolgt anhand von zumindest einem Gravitationssensor, der in der physischen Umgebung angeordnet ist und/oder in einem Detektor (z.B. einem Smartphone) enthalten ist. Mit Hilfe der Gravitationsrichtung, die auf der Gravitationsmarkierung angedeutet wird, und der Position zweier virtueller Einrichtungselemente kann beispielsweise vorhergesagt werden, welche Pumpleistung nötig ist, um einen Fluss von einem virtuellen Einrichtungselement zu einem anderen virtuellen Einrichtungselement zu erzeugen. Darüber hinaus können Lastenverteilungen und/oder Längen von durchhängenden oder auf dem Boden verlaufenden/liegenden Kabeln und Schläuchen berechnet werden.

Gemäß einem Aspekt umfasst das Verfahren ferner einen Schritt eines Bereitstellens wenigstens einer Raum- bzw. Umgebungsmarkierung, insbesondere umfassend eine erste Augmented Reality Markierung (kurz: AR Markierung) und/oder eine QR Markierung (insbesondere nach dem Standard ISO/IEC 18004:2006), in der physischen bzw. realen Umgebung, wobei die räumliche Information, insbesondere einer 3D-Topologie der physischen Umgebung, mittels der Raummarkierung erfasst und wiedergegeben bzw. spezifiziert werden kann.

Eine Augmented Reality (AR) Markierung kann im Wesentlichen jede Art von Markierung sein, die eine Zuordnung bzw. Erkennung ermöglicht. Beispielsweise kann ein Blatt Papier mit einem Punkt oder Kreuz darauf als physische Markierung dienen. Das Blatt Papier kann von einem Verwender an einem Ort in der physischen Umgebung angeordnet werden und die Recheneinheit erkennt, dass ein virtuelles Einrichtungselement an dem Ort virtuell angeordnet werden soll. Auch kann beispielsweise eine Gravitationsrichtung durch eine solche AR Markierung, beispielsweise durch einen Pfeil angedeutet werden. Im Wesentlichen spielt dabei nur eine Rolle, mit welcher Funktion bzw. Kodierung die AR Markierung belegt wird. Die AR Markierung an sich kann jede mögliche Markierung sein, die detektierbar (insbesondere sichtbar) in der physischen Umgebung angeordnet werden kann.

Eine AR Markierung kann nicht nur alleine, sondern beispielsweise auch zusammen mit einem QR Markierung dargestellt werden oder eine solche umfassen. Im Allgemeinen ist zwischen der Kodierung einer QR Markierung und der Kodierung einer AR Markierung zu unterscheiden, da die QR Markierung in Form von schwarzen und weißen Kästchen im Wesentlichen gemäß der entsprechenden QR-Kodierung einem bestimmten String entspricht bzw. zugeordnet ist, der von einer Recheneinheit, der die QR-Kodierung kennt, ausgelesen werden kann. Die QR Markierung enthält im Wesentlichen durch die QR-Kodierung keine kodierte Information über eine Position und/oder Orientierung eines Objektes im Raum enthält. Da aber eine AR Markierung ein beliebiges Muster aufweisen kann, könnte es auch in der Gestalt einer QR Markierung dargestellt sein. Zur Analyse der räumlichen Information würde dann lediglich das Muster an sich anhand der perspektivischen Darstellung bzw. anhand seiner Lage im Raum analysiert werden, wobei die QR-Kodierung zunächst vernachlässigt werden kann. Zum Auslesen der kodierten Information, beispielsweise einer Identität des darzustellenden Objekts, wird die QR-Kodierung jedoch entschlüsselt. Beides kann getrennt voneinander stattfinden. Somit könnte prinzipiell eine QR Kodierung auch als AR Markierung dienen.

Das Bereitstellen oder Anbringen einer Raummarkierung in einem Raum erlaubt dem Verwender, der keine 3D-fähige Kamera besitzt und/oder verwenden möchte, vorteilhaft auf eine einfache Art die Räumlichkeit eine Fläche im Raum zu markieren bzw. anzudeuten, sodass eine Recheneinheit daraus die räumliche Information extrahieren kann. Solche Raummarkierungen können beispielsweise online durch den Vertreiber der Einrichtungselemente zum Ausdrucken bereitgestellt werden. Ein geeigneter Algorithmus, kann dann von der Recheneinheit ausgeführt werden, wodurch die räumlichen Koordinaten oder Merkmale der markierten Fläche abgeleitet bzw. ermittelt werden.

Gemäß einem Aspekt umfasst das Verfahren ferner einen Schritt eines Bereitstellens einer Objektmarkierung (die auch als Bestimmungsort- Markierung eines virtuellen und/oder physischen Einrichtungselementes bezeichnet werden kann und gegebenenfalls auch eine Identitätsmarkierung umfassen kann) , insbesondere umfassend eine zweite Augmented Reality Markierung (kurz: AR Markierung) und/oder eine QR Markierung (insbesondere nach dem Standard ISO/IEC 18004:2006), in der physischen Umgebung, wobei eine virtuelle Position bzw. ein virtueller Zielort und/oder eine virtuelle Orientierung bezüglich der virtuellen Umgebung und/oder eine Identität des virtuellen Einrichtungselements mittels der Objektmarkierung gegeben bzw. spezifiziert ist.

Eine Objektmarkierung kann eine Markierung sein, die ein Verwender in der physischen Umgebung an einem physischen Zielort anordnet, wo ein virtuelles Einrichtungselement an dem Zielort in der virtuellen Umgebung angeordnet werden soll. Die Objektmarkierung kann eine Information über den Zielort und/oder über die Orientierung des virtuellen Einrichtungselements gegenüber der virtuellen Umgebung und/oder über die Identität des virtuellen Einrichtungselements umfassen.

Besonders bevorzugt umfasst die Objektmarkierung eine AR Markierung, welche insbesondere Informationen über deren Position und Lage im Raum der physischen Umgebung umfasst. Diese Information wird dann derart übersetzt, dass ein virtuelles Einrichtungselement anhand der AR Markierung und deren Lage bzw. Positionierung im Raum der physischen Umgebung in einem virtuellen Raum angeordnet werden kann. Insbesondere enthält die AR Markierung auch eine Information über die Identität des virtuellen Einrichtungselements, sodass die Recheneinheit das virtuelle Einrichtungselement identifizieren und entsprechend virtuell in der virtuellen Umgebung anordnen kann. Dieser konkrete Fall könnte dadurch gegeben sein, dass eine AR Markierung und eine QR Markierung physisch an einem Ort in der physischen Umgebung angeordnet werden. Ein sehr spezieller Fall ist gegeben, wenn lediglich eine QR Markierung physisch angeordnet wird, welche dann anhand der Lage ihres Musters im Raum bezüglich der räumlichen Anordnung ausgelesen wird und mittels Entschlüsseln der QR Kodierung auf ihren kodierten Gehalt, beispielsweise eine Identität umfassend, ausgelesen wird.

Gemäß einem Aspekt umfasst das Verfahren folgende Schritte:
- Erlauben eines Auswählens durch einen Verwender und/oder die Recheneinheit zumindest eines virtuellen Einrichtungselements, vorzugsweise aus einer Auswahl mehrerer virtueller Einrichtungselemente, beispielsweise aus einer Produktpalette oder einem Produktkatalog; und insbesondere
- Bereitstellen eines registrierten bzw. bekannten physischen Einrichtungselements in der physischen Umgebung, wobei das Auswählen des zumindest einen virtuellen Einrichtungselements bevorzugt mittels der Recheneinheit, insbesondere automatisch erfolgt und wobei das Auswählen des zumindest einen virtuellen Einrichtungselements auf einem Schritt eines Identifizierens des registrierten physischen Einrichtungselements mit einem virtuellen Einrichtungselement insbesondere aus der Auswahl der mehreren virtuellen Einrichtungselemente basiert.

In anderen Worten kann der Verwender ein gewünschtes virtuelles Einrichtungselement insbesondere aus einer Auswahl, beispielsweise aus einem Online-Produktkatalog selbst auswählen. Zusätzlich oder alternativ kann auch eine Recheneinheit ein geeignetes beispielsweise kompatibles virtuelles Einrichtungselement auswählen. Insbesondere kann eine Recheneinheit eine Mehrzahl geeigneter virtueller Einrichtungselemente vorschlagen, aus der sich der Verwender ein virtuelles Einrichtungselement aussuchen und/oder auswählen kann. Insbesondere kann eine Recheneinheit ein in der physischen Umgebung positioniertes registriertes physisches Einrichtungselement erkennen bzw. identifizieren und einem entsprechenden virtuellen Einrichtungselement zuweisen, was einer automatischen Auswahl des durch das in der physischen Umgebung vorhandene Einrichtungselement vorbestimmten virtuellen Einrichtungselements entspricht.

Die beschriebene Auswahl eines virtuellen Einrichtungselements hat den Vorteil, besonders intuitiv, effizient und anwenderfreundlich zu sein. Die Recheneinheit kann dem Verwender dabei behilflich sein, ein passendes bzw. geeignetes virtuelles Einrichtungselement zu wählen, sodass der Blick in ein Datenblatt nicht mehr zwingend notwendig sein muss. Auch kann die Recheneinheit automatisch registrierte Geräte, also registrierte physische Einrichtungselemente erkennen, die von dem Vertreiber vertrieben werden und/oder in der Vergangenheit vertrieben wurden.

Gemäß einem Aspekt umfasst der zuvor beschriebene Schritt des Identifizierens des registrierten physischen Einrichtungselements einen Schritt eines Erkennens eines oder mehrerer Formmerkmale des Einrichtungselements und/oder einer Identitätsmarkierung, insbesondere einer dritten AR Markierung und/oder QR Markierung des registrierten physischen Einrichtungselements umfasst.

Besonders bevorzugt ist die Ausführungsform, in der eine Recheneinheit anhand typischer bzw. charakteristischer Formmerkmale physischen Einrichtungselements erkennt, um welches registrierte Gerät bzw. physische Einrichtungselement es sich handelt, da vorteilhaft im Wesentlichen kein Input von dem Verwender benötigt wird. Eine andere Ausführungsform basiert auf der Bereitstellung einer Identitätsmarkierung, welche der Verwender vor der Erfassung des Sensordatensatzes an dem entsprechenden physischen Einrichtungselement anbringen kann. Die Recheneinheit wertet diese Identitätsmarkierung, die beispielsweise eine AR Markierung sein kann, aus und ordnet bzw. weist dem physischen Einrichtungselement ein entsprechendes virtuelles Einrichtungselement zu.

Gemäß einem Aspekt umfasst das zumindest eine virtuelle Einrichtungselement zumindest eines der folgenden Einrichtungselemente: einen Bioreaktor, einen Einwegbeutel, ein Behältnis, einen Tank, ein Filtersystem, eine Mischeinrichtung, einen Gärtank, eine Zentrifuge, eine Chromatographiesäule, einen Membran-Adsorber, eine Abfülleinrichtung. Das zumindest eine virtuelle Einrichtungselement umfasst zumindest ein virtuelles Verbindungselement, welches ein virtuelles Kabel oder ein virtueller Schlauch ist. Zum besseren Verständnis der Erfindung kann das virtuelle Verbindungselement zumindest eines der Folgenden umfassen: einen Aufsatz, einen Adapter, eine Verbindung, einen Konnektor, ein Rohr, eine Leitung, eine Röhre, eine Pumpe. Ferner umfasst der Parameterdatensatz insbesondere zumindest eine variable und/oder eine fixe Größe aus den folgenden Parametern: eine Identifizierungskodierung, eine Bestellnummer, ein Volumen, eine Länge, eine räumliche Ausdehnung, einen Durchmesser, eine Struktur, ein Material, einen Betriebsbereich, einen Betriebsgrenzwert, eine Kompatibilität mit einem weiteren virtuellen Einrichtungselement, eine Kompatibilität mit einer biologischen und/oder chemischen Reaktion, einen Parameterdatensatz zu einem Medium, insbesondere einem fluiden Medium, und bevorzugt der biologischen und/oder chemischen Reaktion des Mediums.

Zubehör für biotechnologische und/oder chemische Anwendungen, beispielsweise unter anderem Bioreaktoren, Mehr- und/oder Einwegbeutel, Behältnisse, Tanks, Filtersysteme, Mischtanks und/oder -Einrichtungen, Gärtanks eine Zentrifuge, eine Filtrationssäule, einen Membran-Adsorber, eine Abfülleinrichtung und/oder ähnliche können eine beliebige Komplexität in ihrer Handhabung bzw. Bedienung aufweisen. Oft handelt es sich um sehr spezielle Geräte und Artikel bzw. um solches Zubehör, das für sehr spezielle Zwecke eingesetzt werden kann. Es liegt daher selten auf der Hand, ob verschiedene Geräte miteinander kompatibel sind und aufeinander abgestimmt bzw. harmonisiert werden können. Das beispielhaft genannte Zubehör weist sich im Einzelnen durch die Parametereinträge des jeweiligen Parameterdatensatzes aus. Eine Kontrolle, ob zwei Geräte miteinander kompatibel sind, setzt in der Regel voraus, dass Parametereinträge miteinander verglichen werden. Gemäß dem genannten Aspekt kann dieser Schritt (zumindest teilweise) automatisiert werden, sodass die Recheneinheit vorab schon evaluiert bzw. bewertet bzw. vorhersagt, ob zwei Geräte kompatibel sind. Dazu werden virtuelle Einrichtungselemente erzeugt, beispielsweise virtuelle Bioreaktoren, virtuelle Einwegbeutel, virtuelle Behältnisse, virtuelle Tanks, virtuelle Filtersysteme, virtuelle Mischtanks, virtuelle Gärtanks, die als virtuelle Modelle den jeweiligen physischen Einrichtungselementen entsprechen.

Die beispielhaft benannten virtuellen und/oder physischen Einrichtungselemente können durch Daten- und/oder Parametereinträge gekennzeichnet sein, wie unter anderem ein Volumen, ein Material, eine Druckfestigkeit, eine räumliche Ausdehnung, einen Durchmesser, eine Struktur, einen Betriebsbereich, einen Betriebsgrenzwert, eine Kompatibilität mit einem weiteren virtuellen Einrichtungselement, eine Kompatibilität mit einer biologischen und/oder chemischen Reaktion und/oder einem Sterilisationsverfahren.

Oft handelt es sich bei dem Zubehör um Behältnisse oder das Zubehör umfasst Behältnisse, in denen Medien, beispielsweise Puffersysteme und/oder Zellkulturmedien prozessiert und/oder gelagert werden. Es kann vorkommen, dass ein Medium zwischen zwei Behältnissen fließen soll, beispielsweise falls ein erster Prozess, z.B. die Herstellung eines Stoffs in einem Medium in einem ersten Behältnis erfolgt und anschließend für einen zweiten Prozess in einen zweiten oder weitere Behältnisse erfolgt. Dies setzt voraus, dass ein virtuelles Verbindungselement beide Behältnisse virtuell miteinander verbindet, beispielsweise unter anderem durch einen virtuellen Schlauch, ein virtuelles Rohr, eine virtuelle Leitung, eine virtuelle Röhre, eine virtuelle Pumpe, einen virtuellen Aufsatz, einen virtuellen Adapter, einen virtuellen und/oder physischen Konnektor und/oder eine virtuelle Verbindung, sofern das Medium nicht anderweitig transportiert werden soll. Die genannten Elemente entsprechen dabei insbesondere jeweils einem realen, also physischen Schlauch, einem physischen Rohr, einer physischen Leitung, einer physischen Röhre, einer physischen Pumpe, einem physischen Aufsatz, einem physischen Adapter bzw. einer physischen Verbindung. Derart kann beispielsweise auch vorhergesagt, insbesondere simuliert werden, ob bzw. wie ein Medium, insbesondere eine Flüssigkeit zwischen zwei Behältnissen fließen wird. Es kann insbesondere auch eine benötigte Pumpleistung ermittelt werden, bevorzugt unter Berücksichtigung der Schwerkraft bzw. der Gravitationsrichtung. Darüber hinaus kann es möglich sein, mehrere Prozesse in mehreren Behältnissen vorherzusagen, insbesondere zu simulieren.

Ein virtuelles Verbindungselement ist ein virtuelles Kabel , welches einem physischen Kabel entspricht, oder ein virtueller Schlauch. Beispielsweise kann eine in der physischen Umgebung vorhandene Steckdose von der Recheneinheit erkannt werden und in eine virtuelle Steckdose übersetzt werden. In dem Fall kann ein virtuelles Einrichtungselement, insbesondere ein strombetriebenes Gerät virtuell durch das virtuelle Kabel mit der virtuellen Steckdose verbunden werden. Auch wäre es möglich mehrere virtuelle Einrichtungselemente miteinander beispielsweise durch ein virtuelles Stromkabel und/oder ein virtuelles Datenkabel zu verbinden.

Ein virtuelles Einrichtungselement wird durch die Daten- und/oder Parametereinträge eines Parameterdatensatzes definiert. Ein virtuelles Verbindungselement, wie beispielsweise ein virtueller Schlauch kann durch folgende Dateneinträge eines Parameterdatensatzes unter anderem definiert sein: ein Volumen, eine Länge, ein Gewicht pro Längeneinheit, einen Durchmesser, eine Struktur, ein Material, eine Festigkeit und/oder Flexibilität, einen Betriebsbereich bezüglich Temperatur, Säure- und/oder Basenkonzentration und/oder Druck, einen entsprechenden Betriebsgrenzwert, eine Kompatibilität mit einem anderen virtuellen Einrichtungselement, eine Kompatibilität mit einer biologischen und/oder chemischen Reaktion, eine Resistenz gegenüber einer Sterilisation, eine reaktive/inerte Eigenschaft gegenüber Stoffen.

Ein Daten- und/oder Parametereintrag kann auch eine Funktion bzw. eine Abhängigkeit umfassen. So kann beispielsweise eine im Experiment ermittelte Kennlinie eine Abhängigkeit zwischen zwei Größen bzw. Daten- und/oder Parametereinträgen wiedergeben bzw. darstellen. Beispielsweise können eine Materialfestigkeit und/oder eine Materialausdehnung eines Schlauchs gegenüber eines Drucks aufgetragen sein bzw. durch die Funktion beschrieben sein.

Die Dateneinträge können dazu dienen, beispielsweise einen Fluss eines Mediums zu simulieren, und/oder vorhersagen zu können, welcher virtuelle Adapter oder welches virtuelle Verbindungsstück benötigt wird.

Andere Dateneinträge des Parameterdatensatzes können sich auf ein Medium und/oder einen Prozess beziehen. Solche Dateneinträge charakterisieren zwar nicht die intrinsischen Eigenschaften eines Einrichtungselements, jedoch können sie dazu dienen, einen Prozess und/oder einen Fluss eines Mediums vorherzusagen und insbesondere zu simulieren. Beispielsweise benötigt man zur Ermittlung eines Materialflusses Parameter, wie eine Viskosität und eine Dichte, sowie das Volumen des Mediums. Mit Hilfe dieser Parametereinträge und der Parametereinträge eines virtuellen Schlauchs, wie beispielsweise der Durchmesser und die Länge, kann errechnet werden, wie das Medium durch den Schlauch fließen wird.

Andere Dateneinträge, die administrativen Größen entsprechen und ein virtuelles und damit auch physisches Einrichtungselement charakterisieren, können eine Identifizierungskodierung, eine Bestellnummer, ein Preis pro Stück oder pro Meter und/oder eine vorhandene Stückzahl sein. Diese Parameter können dazu dienen, ein preisliches Angebot zu ermitteln.

Parametereinträge können fixe Einträge, also durch den Verwender, insbesondere einen Käufer unveränderbare Größen umfassen. Beispielsweise wäre dies der Fall bei Größen bzw. Parametereinträgen, wie der Identifizierungskodierung, der Bestellnummer aber auch bei Parametereinträgen, wie Gewicht pro Volumen- oder Längeneinheit, Materialfestigkeit und ähnlichen. Parametereinträge können gleichwohl auch von einem Verwender und/oder einem Käufer bzw. Kunden verändert werden. Beispielsweise kann der Kunde die Länge eines Verbindungselements, das Volumen eines Behältnisses, das Material auf einer Skala mit kontinuierlichen oder diskreten Werten bestimmen.

Gemäß diesem Aspekt wird ein hoher Grad an Flexibilität zur Konfiguration der Einrichtung ermöglicht und zugleich kann eine Vielzahl an Betriebszuständen vorhergesagt, insbesondere simuliert werden. Deshalb bietet diese Ausführungsform einen besonders hohen Grad an Anwenderfreundlichkeit und Effizienz.

Gemäß einem Aspekt weist das Bestimmen der virtuellen Anordnung ein Bestimmen von mindestens zwei virtuellen Einrichtungselementen auf, wobei hiervon ein Einrichtungselement ein virtuelles Verbindungselement aufweist, und das Verfahren ferner folgende Schritte umfasst:
- Prüfen der Kompatibilität zwischen den mindestens zwei virtuellen Einrichtungselementen;
- wobei falls die Kompatibilität in der Prüfung festgestellt wurde:
   ∘ Erlauben eines virtuellen Verbindens der mindestens zwei virtuellen Einrichtungselemente mittels des virtuellen Verbindungselementes durch den Verwender und/oder die Recheneinheit;
- wobei falls die Kompatibilität in der Prüfung nicht festgestellt wurde:
   ∘ Ausgabe einer Fehlermeldung, die das Fehlen der Kompatibilität meldet.

Es besteht insbesondere ein Vorteil darin, vorhersagen zu können, ob zwei physische Einrichtungselemente miteinander kompatibel sind, damit dies nicht erst festgestellt wird, wenn die physischen Einrichtungselemente erworben und dem Kunden angeliefert wurden. Die Kompatibilität wird durch die virtuelle Konfiguration der virtuellen Einrichtungselemente getestet bzw. überprüft. Einer Kompatibilität kann es beispielsweise unter anderem entgegenstehen, wenn zwei Anschlüsse nicht kompatibel wären oder wenn sich Betriebsbereiche, beispielsweise bezüglich des Drucks oder der Temperatur nicht überschneiden würden. Mit der Ausgabe einer Fehlermeldung, welche das Fehlen der Kompatibilität meldet, kann der Verwender und/oder Kunde eine Entscheidung darüber treffen, ob er die physischen Einrichtungselemente, die den gewählten virtuellen Einrichtungselementen entsprechen, tatsächlich erwerben möchte, oder ob er lieber ein kompatibles physisches Einrichtungselement wählen möchte, das seinen Anforderungen entspricht. Die Recheneinheit kann bevorzugt eine Auswahl kompatibler Einrichtungselemente treffen, aus der sich der Verwender ein geeignetes heraussuchen bzw. auswählen kann.

Falls die Prüfung zu dem Ergebnis kommt, dass alle virtuellen Einrichtungselemente miteinander kompatibel sind, so kann sich der Verwender und/oder Kunde gewiss sein, dass er die gewünschten Einrichtungselemente bestellen bzw. erwerben kann, ohne dass ein Risiko besteht, dass diese Einrichtungselemente dann vor Ort nicht miteinander kompatibel sind.

Das Bestimmen der virtuellen Anordnung umfasst ein Bestimmen von mindestens drei virtuellen Einrichtungselementen, wobei eines der mindestens drei virtuellen Einrichtungselemente dem virtuellen Verbindungselement entspricht; wobei der Parameterdatensatz, der dem virtuellen Verbindungselement zugeordnet ist, eine Länge des Verbindungselementes umfasst; und wobei zwei der mindestens drei virtuellen Einrichtungselemente jeweils mindestens einen Anschluss zum Anschließen jeweils eines Endes des virtuellen Verbindungselements umfassen; und das Verfahren ferner folgende Schritte umfasst:
- Bestimmen einer virtuellen Distanz bzw. Entfernung zwischen den mindestens zwei Anschlüssen in der virtuellen Umgebung basierend auf dem Sensordatensatz, dem Parameterdatensatz und der virtuellen Anordnung;
- Bestimmen der virtuellen Länge des Verbindungselementes, insbesondere einer optimalen Länge, insbesondere basierend auf der Distanz zwischen den mindestens zwei Anschlüssen in der virtuellen Umgebung, wobei das Bestimmen der Länge mittels Recheneinheit und/oder mittels Verwender erfolgt.

Bei der Bestimmung der virtuellen Länge erfolgt eine Anordnung des Verbindungselementes in der virtuellen Umgebung unter Berücksichtigung einer detektierten Gravitationsrichtung und der daraus resultierenden Gewichtskraft.

Die beschriebene Ausführungsform hat den Vorteil, dass ein Verbindungselement, insbesondere ein Schlauch auf effiziente und intuitive Weise an die Einrichtung, sowie an die virtuellen Einrichtungselemente und an die Positionierung der Einrichtungselemente in der virtuellen Umgebung angepasst werden kann. Somit kann eine optimale Länge eines Schlauchs abhängig von dessen gewünschten virtuellen Verlauf im Raum bestimmt werden und es kann somit vermieden werden, dass eine zu kurze Länge oder ein Überschuss "auf Verdacht" gewählt wird. Insbesondere bestimmt sich der Verlauf eines Schlauchs realitätsgetreu durch seine eigene Gewichtskraft. Der virtuelle Schlauch wird mit seinen beiden Enden jeweils an zwei virtuellen Einrichtungselementen angeschlossen. Der virtuelle Schlauch hängt von einem virtuellen Anschluss eines virtuellen Einrichtungselements herunter und verläuft virtuell entlang des virtuellen Bodens zu einem anderen virtuellen Anschluss des anderen virtuellen Einrichtungselements.

Das Augmented Reality-System umfasst ferner einen Bildschirm, insbesondere einen touch screen und das Verfahren umfasst ferner einen Schritt einer Abbildung der virtuellen Umgebung und des zumindest einen virtuellen Einrichtungselements in Form einer Augmented Reality-Abbildung der virtuellen Anordnung auf dem Bildschirm.

Eine Benutzeroberfläche (Engl.: "user interface") wird auf dem Bildschirm abgebildet. Diese kann dazu ausgelegt sein von dem Verwender interaktiv genutzt zu werden. Dies bedeutet, dass der Verwender Eingaben machen kann, die von einer Recheneinheit verarbeitet werden. Besonders bevorzugt ist eine Ausführungsform, in der die Benutzeroberfläche intuitiv und einfach gestaltet ist. Insbesondere wird die virtuelle Umgebung, also die rekonstruierte physische Umgebung oder das Abbild der physischen Umgebung zusammen mit ausgewählten und in der virtuellen Umgebung positionierten virtuellen Einrichtungselementen innerhalb der Benutzeroberfläche auf dem Bildschirm dargestellt. Der Verwender kann derart einen visuellen Eindruck während der Verfahrensschritte der virtuellen Konfiguration von der konfigurierten Einrichtung bzw. deren Zwischenergebnissen erlangen.

Gemäß einem Aspekt umfasst das Bestimmen der virtuellen Anordnung ferner mindestens einen Schritt eines Anordnens per "Drag-and-Drop" des zumindest einen virtuellen Einrichtungselements mittels des Verwenders unter Benutzung des Bildschirms.

Diese bevorzugte Ausführungsform erlaubt dem Verwender, besonders intuitiv und effizient unter anderem virtuelle Elemente in einer virtuellen Umgebung zu positionieren, zu verschieben, hinzuzufügen und/oder zu modifizieren und Parameter bzw. Parametereinträge eines Parameterdatensatzes zu bestimmen, zu modifizieren, zu optimieren und/oder zu ändern. Dies kann einerseits per Mauszeiger bzw. cursor und/oder "Drag-and-Drop" erfolgen und insbesondere kann der Verfahrensschritt auf dem touch screen per Hand erfolgen. Zur Ausführung des Schrittes gemäß diesem Aspekt ist kein besonderes Fachwissen oder Vorwissen vorausgesetzt und der Schritt kann in kurzer Zeit intuitiv ausgeführt werden. Unter "Drag-and-Drop" versteht man das Auswählen und das virtuelle Greifen durch ein Anklicken mittels des Mauszeigers und das darauffolgende virtuelle Ziehen des Elements an einen gewünschten Ort in der Benutzeroberfläche auf dem Bildschirm.

Gemäß einem Aspekt kann mindestens eine variable Größe des Parameterdatensatzes, insbesondere eine Länge des virtuellen Einrichtungselements durch den Verwender am Bildschirm, insbesondere durch Betätigung auf dem touch screen, bestimmt werden.

Diese besonders intuitive Ausführungsform erlaubt dem Verwender beispielsweise den möglichen Verlauf eines Schlauches zu bestimmen und/oder vorherzusagen, je nach gewählter Länge. Mittels eines sogenannten "sliders" bzw. "track bars" kann der Verwender beispielsweise per Mausanzeiger oder per Hand auf dem touch screen dynamisch einstellen bzw. bestimmen, wie lang ein virtueller Schlauch sein soll. Alternativ kann ein diskreter Wert und/oder Parametereintrag eingegeben oder angeklickt werden.

Parametereinträge können bevorzugt dynamisch bzw. selektiv eingestellt werden. In anderen Worten kann der Verwender auf einer Skala kontinuierlich oder diskret bestimmen, welchen Wert ein Parametereintrag des Parameterdatensatzes annehmen soll. So kann der Verwender beispielsweise mittels einer "check box" oder mittels eines "sliders" bestimmen, ob das Volumen eines Behälter beispielsweise diskrete Werte, wie etwa 5 I, etwa 100 I, etwa 200 I, etwa 500 I, etwa 1000 I, etwa 3000 I oder etwa 5000 I annehmen bzw. haben soll. Dies macht die Konfiguration besonders einfach und effizient.

Gemäß einem Aspekt umfasst der der physischen Umgebung zugehörige Sensordatensatz mindestens eine der folgenden Größen: eine Temperatur, eine Zeit, eine elektrisches Feldstärke, eine Lichtintensität, eine Vibration, ein Geräusch.

In anderen Worten kann mittels des Sensors bzw. der Sensoreinheit bevorzugt eine Größe bzw. ein dazugehöriges Feld aufgenommen werden, welches beispielsweise mit den räumlichen Koordinaten überlagert werden kann. Falls es sich bei einer der Sensoreinheiten um eine Infrarotkamera handelt, kann der Verwender eine Aufnahme der Temperatur des Raums machen, welche mit der räumlichen Darstellung bzw. der virtuellen Umgebung überlagert wird. Basierend auf einem solchen Datensatz kann der Einfluss einer solchen Größe, wie der Temperatur auf ein Einrichtungselement und/oder einen innerhalb eines Einrichtungselements ablaufender Prozess simuliert werden. Diese besondere Ausführungsform erlaubt dem Verwender, auf einem relativ komplexen Niveau, also unter Einbeziehung einer Vielzahl von Sensorparametern eines Sensorparameterdatensatzes, Prozesse vorab zu simulieren und vorherzusagen, ob die ausgewählten virtuellen Einrichtungselemente in der Realität als physische Einrichtungselemente den Anforderungen der physischen Umgebung sowie der gewünschten Prozesse genügen. Da es sich bei den Produkten, die bei biochemischen Prozessen, verwendet und/oder erzeugt werden, oft um sehr hochwertige Medien bzw. Stoffe handelt, ist es besonders vorteilhaft, wenn ein Prozess zumindest näherungsweise mit Hilfe der verfügbaren Parameter derart modelliert werden kann, dass ein Erfolg bzw. eine Ausbeute eines Prozesses vorab zumindest eingeschätzt oder vorhergesagt, insbesondere simuliert werden kann. Derart ließe sich vermeiden, einen realen Prozess unter hohem Kostenaufwand mit ungeeigneten physischen Einrichtungselementen und/oder Parametern auszuführen.

Auch kann eine Ausführungsform des Verfahrens einen Schritt des Erkennens von Störfaktoren, wie Licht und/oder Vibrationen umfassen, sodass der Verwender darauf hingewiesen werden kann, dass der Störfaktor problematisch für die Verwendung eines physischen Einrichtungselements oder für den Ablauf eines Prozesses sein kann und evtl. durch den Verwender beseitigt werden sollte.

Insbesondere werden Sensorparameter über einen gewissen Zeitraum dynamisch aufgenommen, sodass beispielsweise die Auswirkung einer Temperaturschwankung auf einen Prozess vorhergesagt werden kann.

Gemäß einem Aspekt umfasst der Schritt des Vorhersagens des Betriebszustandes von dem zumindest einen Teil der Einrichtung einen Schritt eines Simulierens zumindest einer dynamischen Größe eines dynamischen Prozesses, und zwar basierend auf dem Sensordatensatz, dem Parameterdatensatz und/oder der virtuellen Anordnung. Der dynamische Prozess umfasst insbesondere zumindest einen aus den folgenden Eigenschaften bzw. Prozessen: einen Materialfluss, einen biologischen Prozess, einen chemischen Prozess, einen physischen Prozess und/oder einen mechanischen Prozess. Es werden deshalb bevorzugt auch Parametereinträge bezüglich eines gewünschten Prozesses bereitgestellt. Beispielsweise kann ein Verwender angeben, welche Menge(n) und/oder welche Substanz(en) für den Prozess verwendet werden. Es kann beispielsweise auch eine Starttemperatur, ein Startdruck und/oder eine Rührgeschwindigkeit eingestellt werden. Das Verfahren kann dazu ausgelegt sein, den Verlauf des Prozesses bezüglich der biochemischen und/oder der thermodynamischen Vorgänge vorherzusagen.

Gemäß einem Aspekt umfasst der Schritt des Vorhersagens eines Betriebszustandes einen Schritt eines Meldens eines korrekten Betriebszustandes oder eines Meldens eines fehlerhaften Betriebszustandes, insbesondere basierend auf dem Schritt des Simulierens der zumindest einen dynamischen Größe.

Es ist vorteilhaft, in einem Modell der virtuellen Einrichtung, die der Verwender insbesondere mit Hilfe einer geeigneten Benutzeroberfläche konfiguriert hat, bereits vorab auszutesten und/oder zu prüfen, ob die entsprechende physische Einrichtung den Anforderungen des Verwenders genügen wird. Falls dies gemäß der Vorhersage und/oder Simulation nicht der Fall sein sollte, kann der Verwender einfach bzw. flexibel virtuelle Einrichtungselemente austauschen und prüfen, ob die neu konfigurierte virtuelle Einrichtung bessere Ergebnisse hinsichtlich der dynamischen Prozesse erzielt. Der Verwender kann alternativ oder zusätzlich auch Parameter eines Parameterdatensatzes anpassen bzw. ändern bzw. variieren.

Das Ausprobieren kann bevorzugt dazu dienen, dass eine virtuelle Einrichtung bezüglich eines gewünschten Gesichtspunktes optimiert wird. Besonders bevorzugt ist dabei der Schritt, des Assistierens durch die Recheneinheit, welche dem Verwender möglicherweise verbesserte und insbesondere optimale Lösungen für die Konfiguration der virtuellen Einrichtung bezüglich eines Gesichtspunktes und insbesondere eines optimalen dynamischen Prozesses vorschlagen kann. Beispielsweise kann die Recheneinheit dem Verwender eine möglichst kostengünstige und/oder platzsparende und/oder effiziente Lösung einer konfigurierten Einrichtung vorschlagen. Auch kann die Recheneinheit einen Vorschlag bezüglich einer optimalen Positionierung der virtuellen Einrichtungselemente in der virtuellen Umgebung machen, indem Flächen erkannt werden, auf denen ein virtuelles Einrichtungselement positioniert werden kann. Dazu kann eine besondere Ausführungsform dem Verwender erlauben, eine oder mehrere Prioritäten zu bestimmen, nach der bzw. denen die Optimierung ausgerichtet sein soll.

Gemäß einem Aspekt umfasst das Verfahren ferner folgende Schritte:
- Erlauben einer Angebotsanfrage für das zumindest eine virtuelle Einrichtungselement basierend auf dem Parameterdatensatz;
- Erstellen eines Angebots basierend auf der Angebotsanfrage;
- Erlauben einer Warenbestellungsanfrage;
- Verarbeitung einer Warenbestellung basierend auf der Warenbestellungsanfrage.

Diese bevorzugte Ausführungsform ermöglicht dem Verwender, insbesondere nach dem Austesten und/oder Testen und Ausprobieren der virtuell konfigurierten Einrichtung, eine Angebotsanfrage zu stellen und ein entsprechendes Angebot für die jeweiligen physischen Einrichtungselemente zu erhalten. Daraufhin kann der Verwender für die gewünschten physischen Einrichtungselemente eine Warenbestellung tätigen. Der Vorteil dieser Ausführungsform besteht unter anderem darin, dass sich der Verwender vor dem Kauf der physischen Einrichtungselemente mit der Einrichtung vertraut machen kann. Insbesondere kann der Verwender und/oder Kunde die virtuelle Einrichtung auf einem Server oder einer anderen Speichereinheit als Datei abspeichern. Dies hat den Vorteil, dass ein Servicemitarbeiter des Anbieters aus einer Entfernung, also nicht vor Ort, online auf das virtuelle Modell der physischen Einrichtung beispielsweise zum Zwecke einer Fehlerdiagnose und/oder technischen Beratung zugreifen kann. Das virtuelle Modell kann somit als sogenannter digitaler Zwilling der physischen Einrichtung dienen. Bei Angabe einer Auslastung der physischen Einrichtung kann insbesondere der Verschleiß alternder Elemente der physischen Einrichtung voraus- und/oder vorhergesagt werden. Darüber hinaus kann der Verwender von dem Vertreiber eine Meldung darüber erhalten, wann eine Inspektion bzw. Wartung der physischen Einrichtung stattfinden sollte.

Gemäß einem Aspekt umfasst das Verfahren einen Schritt einer manuellen bzw. händischen Markierung, bevorzugt per Finger auf einem Touch Screen oder per Mauszeiger, in der virtuellen Umgebung unter Zuhilfenahme eines Bildschirms, insbesondere zur Markierung eines virtuellen Raumpunktes und/oder eines virtuellen und/oder physischen Einrichtungselements.

Falls eine Markierung auch ohne physisches Anbringen einer Raum- und/oder Identitätsmarkierung erfolgen soll, so kann der Verwender auch per Hand in der virtuellen Umgebung nachträglich Markierungen setzen. Beispielsweise kann eine Funktion der Anwendung erlauben, dass man per Finger anzeigen kann, in welche Richtung die Gravitation wirkt und/oder an welchem Ort ein virtuelles Einrichtungselement angeordnet werden soll und/oder welches physische Einrichtungselement identifiziert werden soll. Auch kann der Verwender einen virtuellen Anschluss im virtuellen Raum markieren, beispielsweise eine Steckdose und/oder einen virtuellen Anschluss eines virtuellen Einrichtungselements und/oder einer Gaszufuhr und/oder einen Abfluss. Dies erlaubt dem Verwender mit noch weniger Aufwand eine Einrichtung zu konfigurieren. Insbesondere kann dadurch ein höheres Maß an Flexibilität erzielt werden. Besonders bevorzugt ist eine Funktion der Anwendung, in der ein Schritt des Bereitstellens von Möglichkeiten geboten wird. So kann die Anwendung dem Verwender eine Möglichkeit bereitstellen eine Identitätsmarkierung und/oder eine Raummarkierung per Hand in der virtuellen Umgebung zu setzen.

Die Erfindung betrifft ebenfalls ein Computerprogrammprodukt, insbesondere ein auf einem computerlesbaren Speichermedium gespeichertes Computerprogrammprodukt, für eine computergestützte virtuelle Konfiguration einer Einrichtung, insbesondere einer Bioreaktoreinrichtung, gemäß dem Anspruch 14.

Ein Computerprogrammprodukt kann insbesondere in Form einer Anwendung und/oder App (Engl.: "application") die Recheneinheit dazu veranlassen, die wesentlichen automatisierten Schritte des Verfahrens zur Konfiguration der Einrichtung in der erweiterten Realität auszuführen. Mittels einer Benutzeroberfläche kann die Recheneinheit zusammen mit den interaktiven Eingaben des Verwenders die Konfiguration ausführen. Wesentliche Schritte, die eine Rechenleistung voraussetzen werden dabei zur Unterstützung des Verwenders durch die Recheneinheit ausgeführt.

Ferner betrifft die Erfindung ein Augmented Reality- (AR-) System für eine virtuelle Konfiguration einer Einrichtung, insbesondere eines Bioreaktor-Systems, gemäß dem Anspruch 15.

Ein System für die Erzeugung einer erweiterten Realität, also ein Augmented Reality-System gemäß der Erfindung ist besonders einfach ausgestaltet, sodass vorteilhaft ein durchschnittlicher Verwender, der über ein Smartphone und/oder ein Tablet und/oder einen PC und eine Kamera verfügt, im Wesentlichen keine zusätzlichen Elemente anschaffen und lediglich eine Anwendung und/oder Software herunterzuladen braucht. Gleichwohl kann das Augmented Reality-System besondere Elemente, wie eine 3D-fähige Kamera oder ein Infrarotsichtgerät bzw. eine Infrarotkamera umfassen. Insbesondere wird dem normalen bzw. gewöhnlichen bzw. durchschnittlichen Verwender und/oder Kunden ermöglicht, ohne großen Aufwand eine Einrichtung, insbesondere eine Anlage zur Prozessierung biochemischer und/oder chemischer Medien, insbesondere Flüssigkeiten zu konfigurieren.

Mit physischer Umgebung und physischem Einrichtungselement können auch jeweils eine physikalische bzw. reale Umgebung und ein physikalisches bzw. reales Einrichtungselement gemeint sein. Die Begriffe physisch bzw. physikalisch meinen insbesondere "real" bzw. "reell" bzw. "gegenständlich".

Im Folgenden werden besondere und/oder bevorzugte Ausführungsformen im Detail anhand der Figuren beschrieben. Einzelne Aspekte, die insbesondere nicht in Kombination beschrieben werden, können miteinander explizit kombiniert werden, sofern sie sich nicht gegenseitig ausschließen.

Es zeigen:
**Fig. 1a** ein Flussdiagramm einer chronologischen Abfolge von Verfahrensschritten für die virtuelle Konfiguration einer Einrichtung gemäß einer Ausführungsform;
**Fig. 1b** ein Flussdiagramm einer chronologischen Abfolge von Verfahrensschritten, welche sich den in **Fig. 1a** gezeigten Verfahrensschritten anschließen, für die virtuelle Konfiguration einer Einrichtung 500 gemäß einem Aspekt;
**Fig. 2a** einen Schritt eines Aufnehmens von Sensordaten durch den Verwender gemäß einer Ausführungsform;
**Fig. 2b** einen Schritt einer Digitalisierung der Sensordaten gemäß einer Ausführungsform;
**Fig. 3** einen Schritt eines Bestimmens eines virtuellen Umfelds gemäß einer Ausführungsform;
**Fig. 4** einen Schritt eines Bestimmens eines virtuellen Umfelds gemäß einer Ausführungsform;
**Fig. 5** einen Schritt eines Bestimmens eines virtuellen Umfelds gemäß einer Ausführungsform;
**Fig. 6** einen Schritt eines Bestimmens eines virtuellen Umfelds gemäß einer Ausführungsform;
**Fig. 7** einen Schritt eines Bestimmens eines virtuellen Umfelds gemäß einer Ausführungsform;
**Fig. 8** einen Schritt eines Bestimmens eines virtuellen Umfelds gemäß einer Ausführungsform;
**Fig. 9** einen Schritt eines Vorhersagens eines Betriebszustandes zumindest eines Teils der Einrichtung gemäß einer Ausführungsform;
**Fig. 10** eine Benutzeroberfläche gemäß einer Ausführungsform;
**Fig. 11** eine Benutzeroberfläche gemäß einer Ausführungsform;
**Fig. 12** eine Benutzeroberfläche gemäß einer Ausführungsform;
**Fig. 13** eine Benutzeroberfläche gemäß einer Ausführungsform.

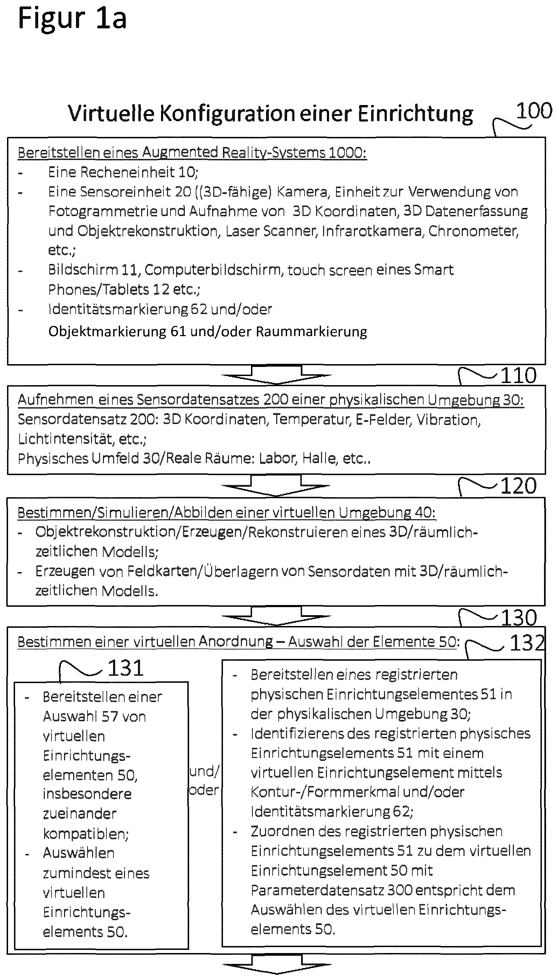

**Fig. 1a** ist ein Flussdiagramm einer im Wesentlichen chronologischen Abfolge von Verfahrensschritten für die virtuelle Konfiguration einer Einrichtung 500 gemäß einer Ausführungsform. Das Verfahren erfolgt insbesondere interaktiv zwischen einer Recheneinheit 10 und einem Verwender 13. In anderen Worten nimmt der Verwender 13 Eingaben vor und die Recheneinheit 10 führt notwendige Rechenprozesse aus und/oder kommuniziert diesbezüglich mit dem Verwender 13. Insbesondere veranlasst eine geeignete Anwendung oder ein Computerprogrammprodukt die Recheneinheit dazu Verfahrensschritte auszuführen. Im Folgenden wird regelmäßig erwähnt, dass eine Recheneinheit 10 einen Verfahrensschritt ausführt. Im Wesentlichen ist deshalb damit gemeint, dass eine Anwendung die Recheneinheit 10 dazu veranlasst, die besagten Verfahrensschritte auszuführen. Im Folgenden werden die Verfahrensschritte zur virtuellen Konfiguration einer Einrichtung 500 gemäß einer Ausführungsform näher beschrieben.

Das Verfahren umfasst einen Schritt eines Bereitstellens 100 eines Systems mit erweiteter Realität bzw. eines Augmented Reality- (AR-) Systems 1000, welches auch als ein "System zur Erzeugung einer erweiterten Realität" bezeichnet wird. Insbesondere wird mittels des Augmented Reality-Systems 1000 eine visuelle Darstellung von Informationen bereitgestellt, die eine Ergänzung von einer Darstellung (z.B. eines Bildes oder Videos) mit computergenerierten Zusatzinformationen oder virtuellen Objekten mittels Einblendung und/oder Überlagerung aufweist. Das Augmented Reality-System 1000 umfasst eine Recheneinheit 10, mindestens eine Sensoreinheit 20 und einen Bildschirm 11 und bevorzugt eine Anwendung bzw. ein Programm bzw. ein Computerprogrammprodukt. Ferner kann das Augmented Reality-System 1000 eine Identitätsmarkierung 62, eine Raummarkierung 33 und/oder eine Objektmarkierung 61 umfassen. Bei der Recheneinheit 10 kann es sich um einen PC und/oder ein Smartphone 12 und/oder ein Tablet 12 und/oder ähnliches handeln. Eine Sensoreinheit 20 umfasst zumindest eine Kamera, insbesondere eine 3D-fähige Kamera bzw. Kamerasystem. Eine 3D-fähigen Kamera ist bzw. umfasst insbesondere eine Einheit zur Verwendung einer Fotogrammetrie bzw. einer Methode, die zur Aufnahme von dreidimensionalen (3D-)Koordinaten bzw. zu der 3D-Datenerfassung ausgelegt ist. Insbesondere ist die 3D-fähige Kamera zur 3D-Datenerfassung und zur Raum- und/oder Objektrekonstruktion ausgelegt. Eine solche Kamera kann dazu beispielsweise einen Laserscanner und eine eigene Recheneinheit zur Digitalisierung der räumlichen Koordinaten umfassen. Jede andere Technologie zur Erfassung von dreidimensionalen bzw. 3D-Koordinaten ist auch denkbar. Zusätzlich oder alternativ kann die Sensoreinheit 20 auch eine Infrarotkamera umfassen. Ferner kann die Sensoreinheit 20 auch dazu ausgelegt sein, ein oder mehrere Umwelteinflüsse und/oder Störsignale und/oder Störgrößen zu erfassen. Beispielsweise kann die Sensoreinheit 20 Geräusche, Vibrationen, Temperaturen und Temperaturschwankungen, elektromagnetische Felder und andere detektieren bzw. aufnehmen, insbesondere über einen zeitlichen Verlauf. Dazu kann eine Sensoreinheit auch eine Uhr bzw. einen Chronometer umfassen. Ein Bildschirm 11 kann ein Computerbildschirm sein und/oder der Bildschirm eines Smartphones 12 und/oder eines Tablets 12, insbesondere mit Touch Screen-fähigkeit. Eine Identitätsmarkierung 62 kann dazu dienen, ein Objekt zu identifizieren. Eine Identitätsmarkierung 62 kann insbesondere eine QR Markierung (insbesondere nach dem Standard ISO/IEC 18004:2006) umfassen. Eine Raummarkierung 33 kann dazu dienen, räumliche Merkmale bzw. 3D-Koordinaten und/oder Richtungen im Raum, beispielsweise eine Richtung der Gravitationskraft, zu bestimmen. Eine Raummarkierung kann ebenfalls insbesondere eine AR Markierung und/oder eine QR Markierung (insbesondere nach dem Standard ISO/IEC 18004:2006) umfassen. Eine Objektmarkierung 61, die physisch in einer physischen Umgebung 30 angeordnet ist, kann hingegen den Ziel- bzw. Bestimmungsort eines virtuellen Einrichtungselements 50 in einer virtuellen Umgebung 40 definieren bzw. bestimmen bzw. festlegen. In einem Augmented Reality-System, welches dazu ausgelegt ist, in Echtzeit eine sich zeitlich ändernde virtuelle Umgebung 40 abzubilden bzw. zu bestimmen, kann eine Objektmarkierung 61 in der physischen Umgebung 30 bewegt und/oder verschoben werden, während der Verwender 13 in Echtzeit überwachen bzw. beobachten kann, wie sich das virtuelle Einrichtungselement 50 virtuell in der virtuellen Umgebung 40 bewegen würde. Derart kann der Verwender 13 sofort bzw. in Echtzeit eine für ihn optimierte virtuelle Anordnung und Positionierung bzw. ein "Zurechtrücken" des virtuellen Einrichtungselements 50 erzielen.

Das Verfahren umfasst einen Schritt des Aufnehmens 110 eines Sensordatensatz 200 einer physischen Umgebung 30 bzw. einer realen Umgebung wie beispielsweise eines Labors. Der Schritt des Aufnehmens 110 eines Sensordatensatz 200 kann insbesondere von dem Verwender 13 ausgeführt bzw. initiiert werden. Dazu bedient sich der Verwender 13 der mindestens einen Funktion der Sensoreinheit 20 bzw. der mindestens einen Sensoreinheit 20.

Der Sensordatensatz 200 umfasst digitalisierte Parameter einer realen physischen Umgebung 30. Besonders bevorzugt umfasst der Sensordatensatz 200 die räumlichen bzw. 3D-Koordinaten von zumindest Teilen der physischen Umgebung 30. Die Digitalisierung bzw. die Übersetzung und/oder Übertragung der räumlichen Merkmale in Dateneinträge des Sensordatensatz 200 kann einerseits mittels der Sensoreinheit, insbesondere der digitalen 3D-fähigen Kamera direkt oder nachträglich mittels der Recheneinheit 10 erfolgen. Ferner kann eine Temperatur und/oder eine Temperaturverteilung im Raum bzw. in der physischen Umgebung 30 beispielsweise mittels einer Infrarotkamera statisch oder dynamisch, insbesondere über einen gewissen Zeitraum aufgezeichnet werden. Die Dateneinträge in den Sensordatensatz 200 können ferner elektrische Felder, magnetische Felder, elektromagnetische Felder, Vibrationen, Lichtintensitäten und/oder andere Einflüsse umfassen.

Eine physische Umgebung 30 entspricht einem real existierenden Raum. Ein solcher Raum kann beispielsweise ein Labor und/oder eine Halle und/oder eine ähnliche Umgebung umfassen. Ein Sensordatensatz 200 kann bevorzugt auch eine Information über die Gravitationsrichtung 33 bzw. die Richtung, in welcher die Gewichtskraft eines potentiellen physischen körperlichen Elementes bzw. Einrichtungselements 51 wirkt bzw. wirken würde, umfassen. Eine solche Angabe kann beispielsweise mittels des Verwenders 13 durch Positionierung einer physischen Markierung 33 zur Angabe der Gravitationsrichtung, beispielsweise in Form eines ausgedruckten Pfeils erfolgen. Alternativ kann die Recheneinheit 10 selbstständig Flächen erkennen und/oder Orientierungen in einem Bild bzw. einer Abbildung erkennen, durch welche die Recheneinheit 10 die Richtung 33 der Gravitationsrichtung und insbesondere eine Gewichtskraft selbstständig ableiten kann.

Darüber hinaus umfasst das Verfahren einen Schritt eines Bestimmens 120, insbesondere eines Simulierens einer virtuellen Umgebung 40 und/oder eines Abbildens der physischen Umgebung 30 in Form einer virtuellen Umgebung 40. Bevorzugt umfasst der Schritt des Bestimmens 120 einen Schritt des Rekonstruierens der aufgenommenen physischen Umgebung 30 anhand der aufgenommenen und digitalisierten Sensordaten des Sensordatensatzes 200. In anderen Worten wird ein räumliches bzw. räumlich-zeitliches Modell der physischen Umgebung 30 modellhaft rekonstruiert. Dies kann beispielsweise auch eine Objektrekonstruktion umfassen, bei der ein dreidimensionales Objekt in der physischen Umgebung 30, beispielsweise ein Tisch oder ein anderes Möbelstück rekonstruiert und im Wesentlichen realitätsgetreu virtuell in der virtuellen Umgebung 40 abgebildet wird.

Ferner kann das Bestimmen 120 auch ein Erzeugen von Feldkarten und/oder das Überlagern von Sensordaten, wie beispielsweise einer räumlichen Temperaturverteilung, mit einer räumlichen 3D-Darstellung der physischen Umgebung 30 umfassen.

Das Verfahren umfasst einen Schritt eines Bestimmens 130, 140 einer virtuellen Anordnung. Dieser Schritt 130, 140 umfasst im Wesentlichen ein Auswählen 130 von mindestens einem virtuellen Einrichtungselement 50.

Der Verfahrensschritt 130 kann einerseits einen Schritt 132 aufweisend ein Bereitstellen einer Auswahl von ein oder mehreren virtuellen Einrichtungselementen 50 umfassen. Insbesondere kann die Recheneinheit eine Vorauswahl von zueinander kompatiblen virtuellen Einrichtungselementen 50 treffen und insbesondere dem Verwender 13 vorschlagen und präsentieren. Gefolgt wird dieser Verfahrensschritt von einem Auswählen zumindest eines virtuellen Einrichtungselements 50. Das Auswählen kann einerseits durch den Verwender 13 oder durch die Recheneinheit 10 erfolgen. Die Recheneinheit kann beispielsweise anhand von vorgegeben Kriterien zumindest teilweise selbstständig auswählen, welches virtuelle Einrichtungselement 50 optimal den Anforderungen des Verwenders 13 genügt.

Zusätzlich oder alternativ kann der Verfahrensschritt 130 auch einen anderen Schritt 132 umfassen. Der Schritt 132 umfasst ein Bereitstellen eines physischen Einrichtungselements 51, welches bereits vorhanden ist bzw. bereits durch den Verwender 13 in der Vergangenheit installiert und/oder angeschafft wurde. Das physische Einrichtungselement 51 wird oder wurde insbesondere von dem Anbieter angeboten und/oder registriert, beispielsweise kann es Dateneinträge eines Parameterdatensatzes zu dem physischen Einrichtungselement 51 in einer Datenbank (z.B. einem Katalog oder einer Produktliste) geben. Bevorzugt ist das physische Einrichtungselement 51 in seiner physischen Erscheinung von dem Sensordatensatz 200 umfasst. In anderen Worten kann das physische Einrichtungselement 51 insbesondere auf einem Bild, bevorzugt einem 3D-Bild, welches einer Abbildung der physischen Umgebung 30 entspricht, zu sehen sein bzw. eingeblendet werden. Der Schritt 132 umfasst ferner ein Identifizieren des registrierten physischen Einrichtungselements 51 mit einem Produkteintrag beispielsweise in einem Katalog und/oder einer Produktliste. Dieses Produkt entspricht dabei einem virtuellen Einrichtungselement 50, also einem Modell eines physischen Einrichtungselements, das von dem Anbieter zum Verkauf geführt wird oder wurde. Das Identifizieren kann einerseits durch das Erkennen einer markanten bzw. charakteristischen Formeigenschaft bzw. eines Formmerkmals mittels der Recheneinheit 10 erfolgen. Dabei werden ein oder mehrere Strukturen bzw. Formmerkmale des physischen Einrichtungselements 51 mit solchen von dem bzw. den virtuellen Einrichtungselement(en) 50 abgeglichen. Alternativ kann die Identifizierung dadurch erfolgen, dass eine von dem Verwender 13 an dem physischen Einrichtungselement 51 angebrachte Identitätsmarkierung 62 von der Recheneinheit erkannt und einem virtuellen Einrichtungselement 50 zugeordnet bzw. zugewiesen wird. Insbesondere kann eine solche Identitätsmarkierung 62 für die angebotenen Produkte durch den Anbieter auf dessen Webseite zum Ausdruck zur Verfügung gestellt sein.

Der Schritt 132 umfasst auch das Zuordnen des registrierten physischen Einrichtungselements 51 zu einem entsprechenden virtuellen Einrichtungselement 50 und zu dem Parameterdatensatz durch den das virtuelle Einrichtungselement 50 und damit auch das physische Einrichtungselement 51 gekennzeichnet ist. Diese Zuordnung entspricht im Wesentlichen einem Auswählen eines virtuellen Einrichtungselements 50, welches bevorzugt automatisch durch die Recheneinheit 10 erfolgt. Es ist zusätzlich oder alternativ auch möglich, dass der Verwender 13 z.B. von Hand bzw. anhand von einer entsprechenden Vorschlagsliste (z.B. in Form eines pulldown Menüs) eine Zuordnung des physischen Einrichtungselements 51 zu einem virtuellen Einrichtungselement 50 durchführt, insbesondere dann, wenn die Recheneinheit 10 das physische Einrichtungselement 51 nicht oder nur fehlerhaft bzw. nicht hinreichend bzw. vollständig identifiziert. Sofern die Recheneinheit 10 das physische Einrichtungselement 51 nicht hinreichend bzw. vollständig identifiziert, kann die Vorschlagliste unter Berücksichtigung der durch die Recheneinheit 10 teilweise erkannten Informationen eingegrenzt bzw. zugeschnitten werden.

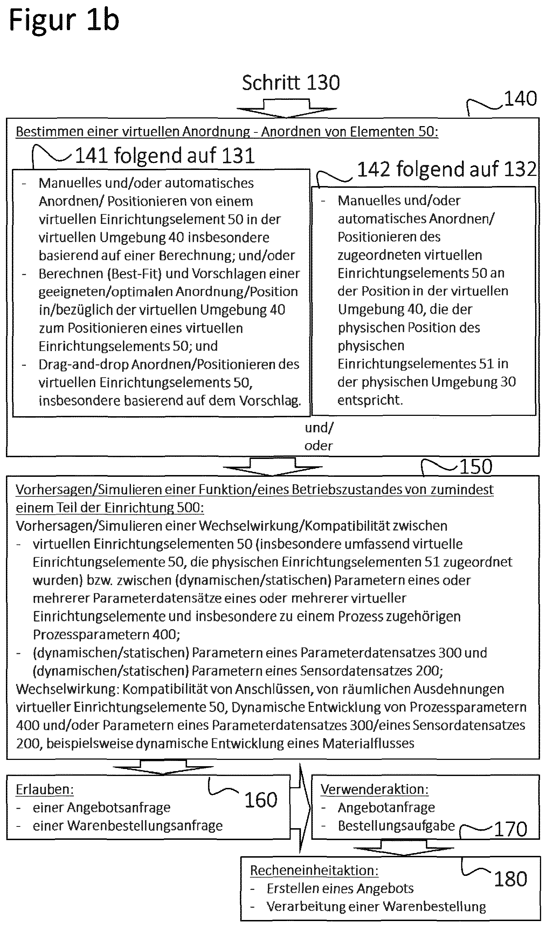

**Fig. 1b** ist ein Flussdiagramm einer chronologischen Abfolge von Verfahrensschritten 140-180, die auf die in **Fig. 1a** dargestellten Verfahrensschritte 100-130 folgen, für die virtuelle Konfiguration einer Einrichtung 500 gemäß einer Ausführungsform. Das Bestimmen einer virtuellen Anordnung, welche bereits teilweise bezüglich der **Fig. 1a** beschreiben wurde, umfasst darüber hinaus einen Schritt eines virtuellen Anordnens 140 bzw. Positionierens von dem zumindest einen virtuellen Einrichtungselement 50, welches im vorherigen Schritt 130 ausgewählt bzw. bestimmt wurde. Auf den Schritt 131, bei dem ein virtuelles Einrichtungselement 50, beispielsweise aus einer vorabgetroffenen Auswahl ausgewählt wird, folgt ein Schritt des manuellen und/oder vorzugsweise automatisierten virtuellen Anordnens 141 bzw. Positionierens des ausgewählten virtuellen Einrichtungselements 50 bezüglich (insbesondere innerhalb) der virtuellen Umgebung 40. Insbesondere kann ein Verwender 13 das ausgewählte virtuelle Einrichtungselement 50 per "Drag-and-Drop" innerhalb der virtuellen Umgebung 40 positionieren und/oder verschieben. Besonders bevorzugt ist dabei, dass die Recheneinheit 10 eine geeignete Position zur Positionierung des virtuellen Einrichtungselements 50 erkennt bzw. bestimmt und dem Verwender 13 entsprechend vorschlägt bzw. anzeigt, sodass der Verwender 13 das virtuelles Einrichtungselement 50 an der vorgeschlagenen virtuellen Stelle der virtuellen Umgebung 40 positionieren kann oder die Positionierung bestätigen kann. Alternativ oder zusätzlich kann die Recheneinheit 10 selbstständig und automatisch das ausgewählte virtuelle Einrichtungselement 50 innerhalb der virtuellen Umgebung 40 positionieren. Insbesondere kann die Recheneinheit 10 selbstständig und automatisch eine Berechnung durchführen, durch welche eine optimale virtuelle Position ermittelt werden kann. Beispielsweise kann die Recheneinheit 10 erkennen, dass es einen virtuellen Ort in der virtuellen Umgebung 40 gibt, welcher frei ist und/oder welcher beispielsweise hinreichend in der Nähe eines Anschlusses (z.B. einer Steckdose) und/oder eines anderen virtuellen Einrichtungselements 50 liegt. Nach der Ermittlung einer geeigneten und/oder optimalen virtuellen Position kann die Recheneinheit 10 das virtuelle Einrichtungselement 50 automatisch an dem ermittelten Ort in der virtuellen Umgebung 40 positionieren.

Das virtuelle Anordnen 140 bzw. Positionieren von dem zumindest einen virtuellen Einrichtungselement 50, welches im vorherigen Schritt 130 ausgewählt bzw. bestimmt wurde, kann zusätzlich oder alternativ auch einen Schritt 142 umfassen, welcher auf den Schritt 132, bei dem ein virtuelles Einrichtungselement 50 ausgewählt bzw. bestimmt wurde, folgt. Der Schritt 142 umfasst ein automatisches Anordnen bzw. Positionieren des virtuellen Einrichtungselements 50 an der virtuellen Position in der virtuellen Umgebung 40, die dem physischen Ort der physischen Umgebung 30 entspricht, an dem das physische Einrichtungselement 51 steht. Dieser Schritt 142 kann beispielsweise lediglich darin bestehen, dass dem identifizierten physische Einrichtungselement 51 ein entsprechender Parameterdatensatz eines entsprechenden virtuellen Einrichtungselements 50 zugewiesen bzw. zugeordnet wird. Der Schritt 142 kann jedoch auch dem Schritt 141 ähneln und ein automatisches Positionieren des zugewiesenen virtuellen Einrichtungselements 50 in der virtuellen Umgebung 40 an dem besagten vorbestimmten virtuellen Ort entsprechen.

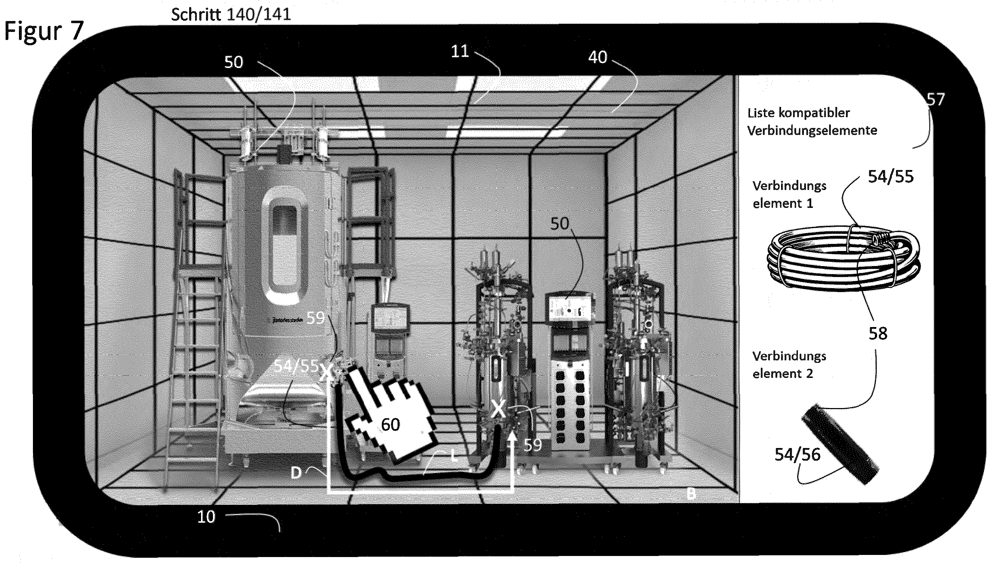

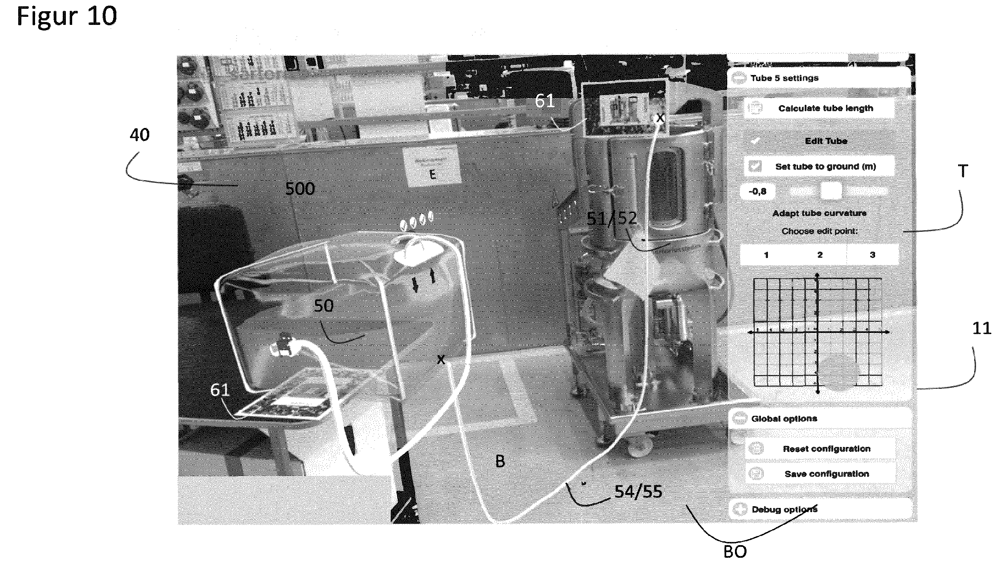

Der Schritt des Bestimmens 130, 140 einer virtuellen Anordnung von virtuellen Einrichtungselementen 50 einer virtuellen Einrichtung 500 kann insbesondere den Schritt umfassen, bei dem zumindest ein virtuelles Einrichtungselement 50 (insbesondere ein Verbindungselement) ausgewählt und virtuell zwischen zwei virtuellen Einrichtungselementen 50 positioniert bzw. geschaltet wird. Diese Schritte umfassen insbesondere das funktionale virtuelle Verbinden zweier virtueller Einrichtungselemente 50, derart, dass eine Verbindung beispielsweise mittels eines virtuellen Schlauchs und/oder eines virtuellen Kabels hergestellt werden kann. Dazu kann die Recheneinheit 10 erkennen, welche Anschlüsse, insbesondere Fluss- und/oder Schlauchanschlüsse und/oder elektrische und/oder Datenübertragungsanschlüsse an einem virtuellen Einrichtungselement 50 oder in der physischen und virtuellen Umgebung 30, 40 vorliegen. Bevorzugt kann die Recheneinheit 10 erkennen, welche Verbindungselemente sich bezüglich der Vorgaben des Verwenders und/oder der virtuellen Einrichtungselemente 50 und/oder der virtuellen Umgebung 40 sich besonders eignen und insbesondere kompatibel mit möglichen virtuellen Anschlüssen der virtuellen Einrichtungselemente 50 sind. Die Recheneinheit 10 kann ferner eine Berechnung anstellen, durch die ermittelt wird, wie lang ein Verbindungselement optimalerweise sein sollte. Dazu kann die Recheneinheit 10 zur Berechnung der optimalen Länge L die ermittelte Gravitationsrichtung und eine entsprechende Gravitationskraft einbeziehen, wenn der Sensordatensatz 200 diese Information enthält. Der Verwender 13 kann zusätzlich oder alternativ die Länge L eines ausgewählten virtuellen Verbindungselements bestimmen und/oder bei Bedarf variieren, wie nachfolgend unter Bezug auf **Fig. 7** und/oder **Fig. 10-12** beispielhaft beschrieben wird.

Auf den Schritt des Bestimmens der virtuellen Anordnung 140 folgt ein Schritt eines Vorhersagens 150, insbesondere einer Simulation, und zwar derart, dass ein Betriebszustand von virtuellen Komponenten bzw. Elementen, insbesondere virtuellen Einrichtungselementen 50 der Einrichtung 500 vorab eingeschätzt und/oder vorhergesagt und/oder simuliert wird. Es kann auch eine Wechselwirkung zwischen virtuellen Komponenten bzw. Elementen, insbesondere virtuellen Einrichtungselementen 50 vorhergesagt und/oder simuliert werden. Ein Betriebszustand kann zusätzlich auch mittels einer Markierung gekennzeichnet werden. Beispielsweise kann eine Markierung darauf hinweisen, dass ein physisches Einrichtungselement 51 außer Betrieb ist. In dem Fall kann dieses physische Einrichtungselement 51 bei der Digitalisierung bzw. der Übersetzung in ein virtuelles Modell im Wesentlichen bezüglich seiner Funktionen vernachlässigt werden.

Es können in diesem Schritt 150 statische und/oder dynamische, also zeitlich abhängige Parameter vorhergesagt und/oder simuliert werden. Bevorzugt kann eine Kompatibilität zwischen zwei virtuellen Komponenten/Elementen, insbesondere virtuellen Einrichtungselementen 50, überprüft bzw. getestet werden. Die Berechnung solche statischen und/oder dynamischen Parameter können den statischen und/oder dynamischen Größen bzw. Dateneinträgen des Sensordatensatzes 200 und/oder des Parameterdatensatzes 300 zugrunde liegen.

Bezüglich des Überprüfens von Kompatibilität(en) kann überprüft werden, ob ein virtuelles Einrichtungselement 50 an einem virtuellen Netzstecker, der einem realen physischen Netzstecker in der physischen Umgebung 30 entspricht, angeschlossen werden kann. Insbesondere ist hier von Interesse, ob die Stromversorgung mit dem virtuellen Einrichtungselement 50 kompatibel ist. In einem anderen möglichen Fall kann überprüft werden, ob eine Gas- und/oder Flüssigkeitszufuhr und/oder -abfuhr geeignet ist, an das virtuelle Einrichtungselement 50 angeschlossen zu werden, beispielsweise, ob die Anforderungen und Gegebenheiten bezüglich der Größen, wie Druck und/oder Strom kompatibel sind. In wieder einem anderen Fall kann überprüft werden, ob zwei virtuelle Einrichtungselemente 50 miteinander kompatibel sind oder zumindest miteinander harmonisiert oder aufeinander abgestimmt werden können (z.B. mittels geeigneter zwischenzuschaltenden Komponenten), sodass diese miteinander verbunden werden können. Beispielsweise kann überprüft werden, ob die Betriebsbereiche zweier virtueller Einrichtungselemente 50 bezüglich einer Größe bzw. eines Parameters nicht miteinander überlappen bzw. übereinstimmen, wenn dies erforderlich sein sollte. Dies kann der Fall sein, wenn ein virtuelles Einrichtungselement 50 innerhalb eines bestimmten Betriebs-Druckbereiches betrieben werden kann, der nicht zumindest teilweise mit dem Betriebs-Druckbereich eines anderen virtuellen Einrichtungselement 50 überlappt bzw. übereinstimmt, wobei eine offene Verbindung zwischen beiden virtuellen Einrichtungselementen 50 besteht. Ein weiteres Beispiel kann sein, dass ein virtuelles Einrichtungselement 50 an eine virtuelle Steckdose angeschlossen werden soll, die einen falschen Spannungswert und/oder einen nicht ausreichenden Stromwert aufweist. Das Überprüfen einer Kompatibilität kann auch lediglich die Kompatibilität von Anschlüssen und/oder die räumliche Ausdehnung bezüglich einer möglichen Positionierung in der virtuellen Umgebung 40 virtueller Einrichtungselemente 50 betreffen. In anderen Worten kann beispielsweise überprüft werden, ob ein virtuelles Einrichtungselement 50 in der virtuellen Umgebung 40 ausreichend Platz zur Positionierung hat.

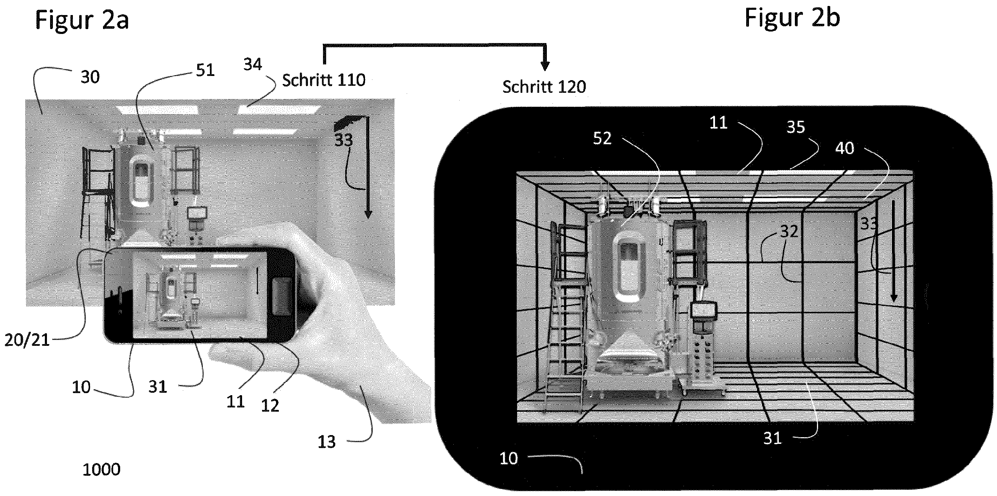

Der Schritt des Vorhersagens 150 eines Betriebszustandes kann ferner den Schritt einer Simulation eines dynamischen Prozesses bzw. dynamischer Prozessparameter 400 und/oder eines Materialflusses zwischen zwei virtuellen Einrichtungselementen 50 umfassen. Prozessparameter 400 können zuvor von einem Verwender und/oder aus einer Datenbank bestimmt und der Recheneinheit 10 übermittelt werden. Beispielsweise kann ein Verwender 13 bestimmen, dass er innerhalb eines virtuellen Einrichtungselements 50 eine bestimmte biochemische Reaktion ablaufen lassen möchte. Dazu kann der Verwender 13 die Art des Prozesses bzw. eine bestimmte Reaktion, die angestrebt wird, die Menge des gewünschten Edukts und/oder Produkts, die Temperatur und/oder dessen zeitlicher Verlauf, und ähnliches als jeweilige (z.B. in eine Tabelle bzw. in einen Datensatz einzugebenden) Prozessparameter 400 bestimmen bzw. festlegen. Anhand der der Recheneinheit 10 zur Verfügung stehenden Daten, insbesondere unter anderem die Prozessparameter 400, den Sensordatensatz 200 und den Parameterdatensatz 300 kann eine Simulation eines biochemischen und/oder chemischen und/oder thermodynamischen und/oder biologischen Prozesses erfolgen. Dazu werden bekannte Zusammenhänge, beispielsweise Abhängigkeiten verschiedener Parameter zur Simulation herangezogen. Eine Simulation kann abhängig von der Anzahl einzubeziehender Parameter beliebig komplex sein oder lediglich einer sehr groben Näherung entsprechen. Die virtuelle Umgebung zusammen mit der Simulation bzw. der Vorhersage entspricht einem beliebig komplexen Modell der Realität, welche eintreten würde unter den vorherbestimmten Umständen, die sich durch die gegebenen Parametereinträge auszeichnen. Insbesondere kann die Vorhersage äußere Einflüsse, die ein oder mehreren Einträgen in einem Sensordatensatz 200 entsprechen, in der Simulation berücksichtigen. Beispielsweise kann eine reale Lichtquelle 34, wie sie in **Fig. 2a** gezeigt ist, und/oder eine Temperaturschwankung eine Auswirkung auf einen Prozess haben, die mittels der Simulation vorhergesagt werden kann. Dies wäre beispielsweise der Fall, wenn eine Lichtquelle, beispielsweise ein Fenster, durch das Tageslicht tritt, den Prozess einer Photosynthese eines biologischen Mediums beeinflussen würde. Auch kann eine typische Temperaturschwankung in der physischen Umgebung 30, beispielsweise zwischen Tages- und Nachtzeiten und/oder zwischen Winter- und Sommerzeiten, welche insbesondere zuvor mittels der Sensoreinheit 20 gemessen bzw. aufgezeichnet wurde, eine Auswirkung auf den Prozess bzw. die Reaktion haben. Solche Einflüsse und deren Auswirkungen, die für den Verwender 13 von Interesse sind, können durch die Simulation zumindest näherungsweise vorhergesagt bzw. abgeschätzt werden. Dabei soll im Wesentlichen das Ziel verfolgt werden, eine optimale Konfiguration der Einrichtung bzw. Anlage 500 vorab zu erreichen. Beispielsweise kann die Recheneinheit 10 bei der Detektion von störenden Lichtquellen und/oder Temperaturschwankungen und entsprechender nicht zufriedenstellender simulierter Prozessergebnisse, weitere Produkte vorschlagen, die diese vorhergesagten Auswirkungen unterdrücken können. Solche Produkte bzw. physische Einrichtungselemente können zum Beispiel umfassen: ein abdunkelndes Element, beispielsweise ein Vorhang oder ein Schirm, wodurch Licht störender Lichtquellen abgefangen werden kann und/oder ein Thermostat zur aktiven Ausgleichung von äußeren Temperaturschwankungen. Solche Produkte können auch bereits in ein physisches Einrichtungselement 51 integriert bzw. integrierbar sein und als besondere Ausstattung mitgeliefert werden.

Der Schritt des Vorhersagens 150, insbesondere der Simulation kann gefolgt werden von der Übermittlung einer Statusmeldung an den Verwender 13 und/oder auf den Bildschirm 11, insbesondere auf die Benutzeroberfläche, ob die Einrichtung 500 im Wesentlichen fehler- und einwandfrei betrieben werden kann und insbesondere ob ein Prozess einen gewünschten Erfolg erzielt. Eine Fehlermeldung würde hingegen indizieren, dass grundlegende Probleme vorhergesagt werden oder aus der Simulation folgen. Das Ergebnis der Simulation kann auch bereits Probleme vorab kenntlich machen und darauf hinweisen, dass eine besondere Ausstattung und/oder alternative physische Einrichtungselemente 51 zum einwandfreien Betreiben der Einrichtung 500 benötigt werden. Insbesondere kann ein Verwender 13 ein virtuelles Modell einer selbstkonfigurierten Einrichtung 500 bereits vorab ausprobieren bzw. testen und/oder sich mit den Funktionen vertraut machen.

Im Anschluss an die Übermittlung einer Statusmeldung kann der Verwender 13 entscheiden, ob er ein oder mehrere physische Einrichtungselemente 51, die den entsprechenden virtuellen Einrichtungselementen 50 der konfigurierten Einrichtung 500 entsprechen, erwerben möchte. Dazu erlaubt das Verfahren in einem Schritt 160, dass der Verwender 13 eine Angebotsanfrage und möglicherweise eine Warenbestellungsanfrage bzw. Bestellungsaufgabe an einen oder mehrere Anbieter übermitteln kann. In einem anderen Schritt 170 tätigt der Verwender 13 die entsprechenden Aktionen, und zwar die Angebotsanfrage und die Bestellungsaufgabe. Dieser Schritt kann beispielsweise die Eingabe persönlicher Daten des Verwenders 13 umfassen. Im Schritt 180 wird die Angebotsanfrage verarbeitet, worauf ein Angebot erstellt wird. Ferner wird die Bestellungsaufgabe entsprechend des Angebots und insbesondere der persönlichen Daten des Verwenders 13 verarbeitet und möglicherweise an den Vertreiber bzw. Verkäufer bzw. die Sales Unit weitergeleitet und verarbeitet.

Insbesondere kann das Verfahren entsprechend der **Fig.1a** und 1b zur individuellen Beratung durch einen Vertreter bzw. Verkäufer und/oder Servicepersonal begleitend eingesetzt werden. Das Modell der konfigurierten Einrichtung 500 kann darüber hinaus in einem Datenspeicher abgelegt werden. Bei Bedarf, insbesondere zur Fehleranalyse kann ein Servicemitarbeiter auf das Modell (z.B. online) zugreifen und mögliche Schwachstellen bzw. Fehler diagnostizieren. Falls der Verwender 13 ein Logbuch, insbesondere ein digitales Logbuch über den Betrieb der physischen erworbenen Einrichtung bzw. Anlage führt, kann dieser Datensatz des Logbuchs in eine Anwendung geladen werden, die den Betrieb der Einrichtung 500 anhand des gespeicherten Modells simuliert und z.B. auf einen möglichen Verschleiß aufmerksam machen kann. Ferner kann eine automatisierte Meldung an der Verwender 13 abgeschickt werden, wenn eine Wartung und/oder Reparatur und/oder Inspektion der Einrichtung 500 benötigt wird. In diesem Fall wird das Modell im Wesentlichen auch als sogenannter "digitaler Zwilling" verwendet.

Die Fig. 2a verbildlicht einen Verfahrensschritt gemäß einer Ausführungsform, der im Rahmen von Schritt 110 in Fig. 1a ausgeführt wird, und insbesondere im Wesentlichen dem Schritt 110 in Fig. 1a entspricht. Mittels eines Augmented Reality-Systems 1000 kann der Verfahrensschritt 110 ausgeführt werden. Ein beispielhaftes Augmented Reality-Systems 1000 weist ein Smartphone 12 auf. Es wird in diesem Schritt 110 durch den Verwender 13 eine Fotografie 31 bzw. Abbildung einer physischen Umgebung 30, insbesondere eine Fotogrammetrie einer physischen Umgebung 30 mittels eines Detektors (z.B. mittels einer Kamera 21 eines Smartphones 12) aufgenommen. Dieser Schritt kann alternativ auch automatisiert und durch eine Recheneinheit 10 ausgeführt werden. Die Abbildung 31 der physischen Umgebung 30 ist auf dem Display bzw. Bildschirm 11 des Smartphones 12 zu erkennen. Das Smartphone 12 umfasst eine Recheneinheit 10 bzw. steht mit dieser in Verbindung und einen Bildschirm bzw. Display 11, insbesondere einen berührungsempfindlichen Bildschirm (bzw. Touch Screen) und eine Sensoreinheit 20, die die Kamera 21 umfasst. Die Ausführung der Rechenschritte, insbesondere der einfachen, wenig komplexen Rechenschritte, kann teilweise mittels der Recheneinheit 10 ausgeführt werden. Kompliziere Rechenschritte, die eine hohe Rechenleistung erfordern können zumindest teilweise mittels einer externen Recheneinheit bzw. einem Cluster ausgeführt werden, mit dem das Smartphone 12 verbindbar ist. Ergebnisse dieser Rechenschritte werden dann lediglich an das Smartphone 12 übermittelt. Auf das Smartphone 12 kann eine entsprechende Anwendung (bzw. App) geladen sein und in Zusammenhang mit bzw. während des Verfahrens ausgeführt werden. Die Anwendung kann zusammen mittels einer geeigneten Benutzeroberfläche insbesondere dazu ausgelegt sein, den Verwender 13 durch die Konfiguration einer Einrichtung zu leiten bzw. führen und/oder auffordern, ein oder mehrere Eingaben vorzunehmen.

In der physischen Umgebung 30 ist eine Markierung zur Angabe einer Gravitationsrichtung 33 positioniert, welche auch in der Abbildung 31 der physischen Umgebung 30 abgebildet wird. Diese Markierung kann lediglich in einem Pfeil bestehen, der die Richtung der Gravitation impliziert. Erfindungsgemäß wird die Gravitationsrichtung mittels eines Sensors bestimmt; sie kann mittels eines Sensors des Smartphones 12 bestimmt werden.

In der physischen Umgebung 30 ist ferner ein physisches Einrichtungselement 51 positioniert. Bei dem physischen Einrichtungselement 51 handelt es sich um eine Bioprozessanlage, die bereits in einer Produktdatenbank registriert ist und von der Recheneinheit 10 erkannt und identifiziert werden kann. Falls das physische Einrichtungselement 51 einer Komponente entspricht, die nicht registriert ist, so kann die räumliche Struktur erfasst und digitalisiert bzw. in ein dreidimensionales virtuelles Modell übersetzt werden.

**Fig. 2b** verbildlicht einen Verfahrensschritt gemäß einer Ausführungsform, der im Rahmen von Schritt 120 in **Fig. 1a** ausgeführt wird, und insbesondere im Wesentlichen dem Schritt 120 in **Fig. 1a** entspricht. Dieser Schritt kann zumindest in Teilen auch in dem Schritt 110 enthalten sein. In anderen Worten werden zwei Schritte 110 und 120 separat beschrieben, die gleichwohl in einem Schritt bzw. zumindest teilweise zur gleichen Zeit ablaufen können und/oder im Wesentlichen miteinander verknüpft sein können.

In der **Fig. 2b** ist ein Smartphone oder Tablet 12 gezeigt, auf dessen Bildschirm bzw. Display 11 die Abbildung 31 der physischen Umgebung 30 gemäß einer Ausführungsform zu sehen ist. Das Smartphone oder Tablet 12 ist nicht zwingend das Gerät, mit dem die Sensordaten 200 aufgenommen wurden. Es kann beispielsweise auch ein Rechner bzw. Personal Computer (PC) sein, auf den die Sensordaten 200 geladen bzw. transferiert werden. Die Abbildung 31 der physischen Umgebung 30 ist im Wesentlichen ein Bestandteil des Sensordatensatzes 200.

Es kann bevorzugt sein, dass in dem Schritt 120 der Sensordatensatz 400 zusammen mit der Abbildung 31 der physischen Umgebung 30 derart verarbeitet werden, dass eine virtuelle Umgebung 40 generiert wird. Es kann darüber hinaus auch sein, dass die virtuelle Umgebung 40 bereits kurz nach dem Aufnehmen des Sensordatensatzes 200, also im Schritt 110, generiert bzw. erzeugt wird mittels einer Recheneinheit, die in einer digitalen Kamera integriert ist. Alternativ kann auch die Abbildung 31 der physischen Umgebung 30 bereits im Wesentlichen einer virtuellen Umgebung 40 entsprechen. Es kann zudem auch sein, dass eine räumliche Information, beispielsweise die 3D-Koordinaten einer Fläche der physischen Umgebung 30 durch eine Raummarkierung, die an der Fläche der physischen Umgebung 30 angeordnet wird, angedeutet bzw. gegeben wird. Erst durch Auswertung der Lage der Raummarkierung kann diese Information über den Raum digitalisiert werden. Ganz ähnlich kann eine Objektmarkierung 61 (oder mehrere) an oder auf einer Fläche der physischen Umgebung 30 angeordnet werden. Die physische Fläche in der physischen Umgebung kann dabei einerseits in eine virtuelle Fläche in der virtuellen Umgebung 40 übersetzt werden. Jedenfalls wird das der Objektmarkierung 61 entsprechende virtuelle Einrichtungselement 50 an dem durch die Objektmarkierung 61 definierten virtuellen Ort bezüglich der virtuellen Umgebung 40 positioniert und/oder ausgerichtet und/oder dargestellt.

Es sei diesbezüglich zudem erwähnt, dass das erfindungsgemäße Verfahren im Allgemeinen nicht zwingend erfordert, dass eine physische Umgebung 30 auf einem Bildschirm 11 abgebildet wird. In anderen Worten kann das Verfahren alternativ nicht dem "What-you-see-is-what-you-get-Prinzip" unterliegen. Das Verfahren kann in dem Fall auch recht abstrakt verlaufen, beispielsweise über einen Texteditor, in dem der Verwender einen Quellcode eingeben und/oder bearbeiten kann, anhand dessen die Verfahrensschritte verarbeitet und/oder ausgeführt werden können.

Die in **Fig. 2a** gezeigte Lichtquelle 34 kann in der virtuellen Umgebung der **Fig. 2b** als funktionales Element und zwar als virtuelle Lichtquelle 35, welche ein virtuelles Licht aussendet, erkannt bzw. rekonstruiert werden.

In **Fig. 2b** ist auf dem Bildschirm bzw. Display 11 nicht lediglich die Abbildung 31 der physischen Umgebung 30, sondern eine virtuelle Umgebung 40 gezeigt, die als digitales Modell der physischen Umgebung 30 dient. Durch das eingezeichnete Raster 32 wird angedeutet, dass die dreidimensionalen Eigenschaften der physischen Umgebung 30, also die 3D-Koordinaten digitalisiert wurden.

Bevorzugt kann die räumliche Struktur eines physischen Einrichtungselements 51 in der virtuellen Umgebung 40 abgebildet werden. Insbesondere handelt es sich bei dem physischen Einrichtungselement 51 um ein bekanntes bzw. registriertes. Auch können räumliche Strukturen ein oder mehrerer anderer nicht registrierter physischen Einrichtungselemente 51 oder Komponenten, wie unter anderem Tische, Laborbänke, Steckdosen und/oder Gaszufuhren oder ähnliches in der virtuellen Umgebung 40 abgebildet werden. Diese sind im Schritt 120 als 3D-rekonstruierte Objekte bzw. Abbildungen 52 physischer Einrichtungselemente 51 in der virtuellen Umgebung sichtbar. Im Schritt 120 ist die Abbildung 52 eines registrierten physischen Einrichtungselements 51 zunächst unidentifiziert, d.h. die Recheneinheit hat noch nicht erkannt, um welches physische Einrichtungselement 51 es sich handelt und es wurde noch keine Zuweisung zu einem virtuellen Einrichtungselement 50 vorgenommen.

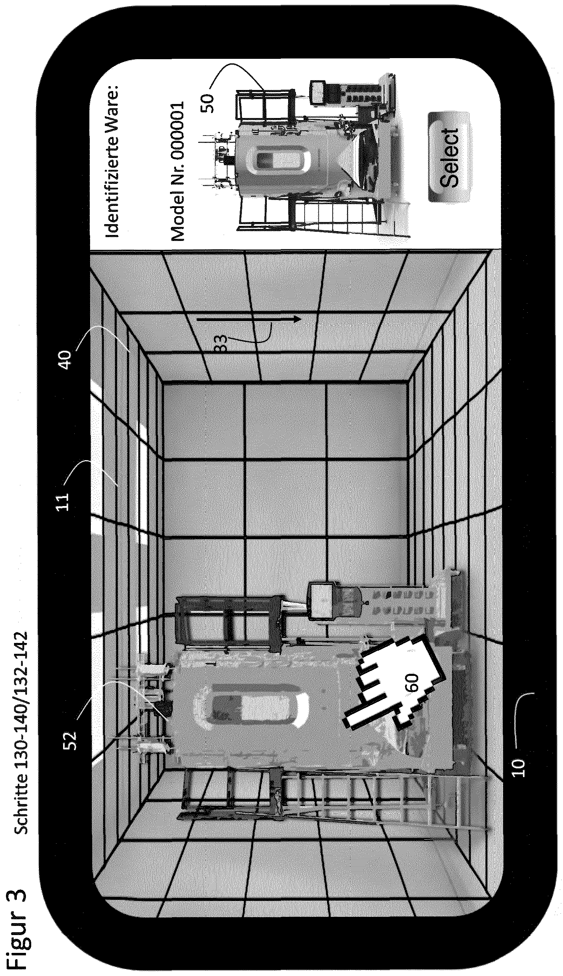

In **Fig. 3** werden die Verfahrensschritte 130 bis 140, insbesondere die Schritte 132 bis 142 gemäß einer Ausführungsform zumindest teilweise näher dargestellt. Die Recheneinheit 10 erkennt in diesem Schritt, dass es sich bei der Abbildung 52 des physischen Einrichtungselements 51 um ein bekanntes, insbesondere registriertes handelt. In diesem Fall kann das physische Einrichtungselement 51 mit Produkten eines Produktkatalogs oder einer Produktliste abgeglichen werden. Ein Produkt kann beispielsweise als virtuelles Einrichtungselement 50 digital in einer Datenbank vorliegen. Mindestens kann das Produkt jedoch einem Parameterdatensatz zugeordnet sein. Beispielsweise kann der Parameterdatensatz des entsprechenden Produkts auch 3D-Koordinaten der räumlichen Struktur des Produkts umfassen. Diese 3D-Koordinaten der räumlichen Struktur des Produkts können dann mit den 3D-Koordinaten der Abbildung des physischen Einrichtungselements 51 abgeglichen werden. Falls es ein oder mehrere Übereinstimmungen in den strukturellen Merkmalen zwischen physischem Einrichtungselement 51 und Produkt gibt, so kann die Recheneinheit das physische Einrichtungselement 51 anhand der Abbildung 52 erkennen und einem Produkt, insbesondere einem virtuellen Einrichtungselement 50, mindestens jedoch einem Parameterdatensatz 300 zuordnen. Dieser Schritt entspricht der automatischen Identifizierung eines physischen Einrichtungselements 51.

Der besagte Schritt des Identifizierens 120 kann unter Assistenz durch einen Verwender 13 ausgeführt werden, z.B. wenn die Recheneinheit 10 die Identifikation nicht durchführen konnte und/oder nicht automatisch nach einem zu identifizierenden registrierten physischen Einrichtungselement 51 suchen soll, weil dies beispielsweise eine zu hohe Rechenleistung erfordern würde. Dies ist beispielhaft in der **Fig. 3** angedeutet. In dem Fall kann der Verwender 13 per Anzeiger 60 (z.B. Mauszeiger 60 und/oder mittels Berührung auf dem berührungsempfindlichen Bildschirm) anzeigen, wo ein mögliches zu identifizierendes physisches Einrichtungselement 51 in der virtuellen Umgebung 40 abgebildet ist. Einerseits kann die Recheneinheit 10 durch das Anzeigen des Verwenders 13 veranlasst werden, selbst einen Suchlauf zu starten, welcher insbesondere dazu führt, dass automatisch ein physisches Einrichtungselement 51 identifiziert wird. Andererseits kann die Recheneinheit 10 eine Auswahl möglicher Produkte, insbesondere virtueller Einrichtungselemente 50 dem Verwender 13 anzeigen, wobei die virtuellen Einrichtungselemente 50 ähnliche strukturelle räumliche Merkmale zu denen des physischen Einrichtungselements 51 aufweisen. Beispielsweise kann es sich um verschiedene Generationen und/oder Baureihen und/oder Serien eines äußerlich ähnlich oder gleich erscheinenden Produkts handeln. In dem Fall kann der Verwender 13 das zutreffende virtuelle Einrichtungselement 50 aus der vorgeschlagenen Auswahl (z.B. als Liste mit ein oder mehreren icons bzw. Darstellungen und/oder als drop-down Menü) selbst bestimmen indem er es beispielsweise anklickt bzw. auswählt und/oder einen "Select-Button", wie in der **Fig. 3** angedeutet ist, anklickt.

Durch das Zuordnen des physischen Einrichtungselements zu einem virtuellen Einrichtungselement 50 bzw. nach dieser Zuordnung erfolgt ein Anordnen 140, insbesondere ein automatisches Anordnen 142 des virtuellen Einrichtungselements 50 an der virtuellen Position der Abbildung 52 des physischen Einrichtungselements 51. Andererseits kann auch ein virtuelles Einrichtungselement 50, das dem physischen Einrichtungselement 51 entspricht, in der virtuellen Umgebung der Abbildung 52 des physischen Einrichtungselements 51 überlagert und/oder damit ausgetauscht werden. Bevorzugt ist es möglich, das virtuelle Einrichtungselement 50, das dem physischen Einrichtungselement 51 entspricht, und/oder die Abbildung 52 des physischen Einrichtungselements 51 in der virtuellen Umgebung zu verschieben und/oder zu drehen und/oder anders anzuordnen bzw. zu positionieren.

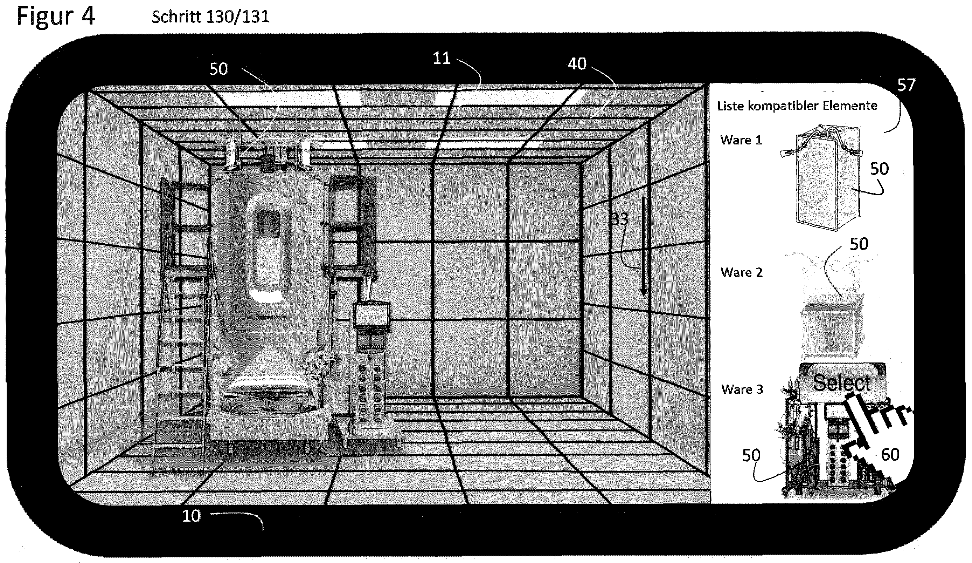

In **Fig. 4** wird der Verfahrensschritt 130, insbesondere der Schritt 131 gemäß einer Ausführungsform näher dargestellt. Die Recheneinheit 10 bietet auf der Benutzeroberfläche, welche auf dem Bildschirm 11 abgebildet wird, eine Liste 57 von ein oder mehreren virtuellen Einrichtungselementen 50 an, aus denen der Verwender 13 ein virtuelles Einrichtungselement 50 auswählen bzw. bestimmen kann. Insbesondere trifft die Recheneinheit 10 eine Vorauswahl von virtuellen Einrichtungselementen 50 bezüglich eines Gesichtspunktes, welcher durch den Verwender 13 vorbestimmt werden kann. Beispielsweise kann eine Liste 57 von virtuellen Einrichtungselementen 50, welche beispielhaft durch "Ware 1", "Ware 2" und "Ware 3" angedeutet sind, angezeigt werden, wobei diese kompatibel miteinander und/oder dem bereits identifizierten physischen Einrichtungselement 51 bzw. virtuellen Einrichtungselement 50 zugeordnet sind.

Der Verwender 13 kann mit dem Anzeiger (z.B. Mauszeiger) 60 ein entsprechendes virtuelles Einrichtungselement 50 aus der Liste 57 wählen, indem er den angezeigten Artikel bzw. einen dazugehörigen "Select-Button" anklickt. Alternativ kann auch die Recheneinheit 10 ein entsprechendes virtuelles Einrichtungselement 50 aus der Liste 57 auswählen, insbesondere, wenn es ein virtuelles Einrichtungselement 50 ist, das in der Einrichtung 500 zur einwandfreien Funktion notwendig ist. Beispielsweise kann ein Verwender 13 eine virtuelle Pumpe aus einer Liste 57 bzw. einem Sortiment bzw. Produktkatalog auswählen. Die Recheneinheit 10 kann dadurch veranlasst werden, gleich ein passendes Öl für die der virtuellen Pumpe entsprechenden physischen Pumpe auswählen, sodass die physische Pumpe nach dem Kauf und der Anlieferung sofort mit dem Öl befüllt werden und in Betrieb genommen werden kann.

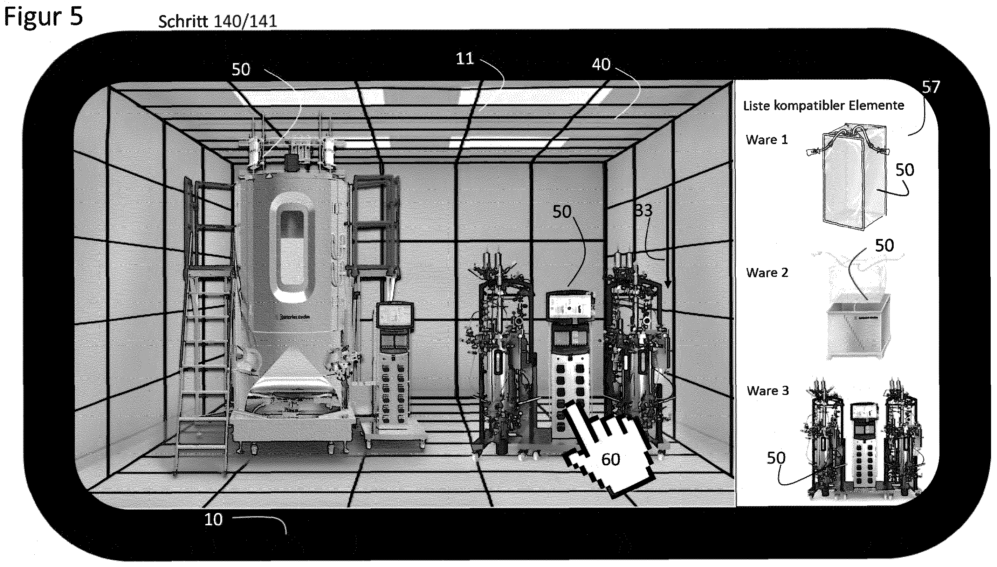

In **Fig. 5** wird der Verfahrensschritt 140, insbesondere der Schritt 141 gemäß einer Ausführungsform näher dargestellt. Der Verwender 13 kann mittels des Anzeigers (z.B. Mauszeigers) 60 das ausgewählte virtuelle Einrichtungselement 50 per "drag-and-drop" an einen Ort innerhalb der virtuellen Umgebung 40 bzw. bezüglich der virtuellen Umgebung 40, insbesondere unter Einbeziehung der Gewichtskraft bzw. der Gravitationsrichtung 33, anordnen. Denkbar ist auch eine Eingabe über den Touch Screen beispielsweise mittels des Fingers. Der Verwender 13 kann die virtuellen Einrichtungselemente 50 in der virtuellen Umgebung 40 verschieben und/oder drehen und/oder neu anordnen.

Insbesondere kann die Recheneinheit 10 eine optimale Anordnung der virtuellen Einrichtungselemente 50 bezüglich eines speziellen (vorbestimmten bzw. vorbestimmbaren) Gesichtspunktes berechnen und/oder vorschlagen und/oder vornehmen. Beispielsweise kann eine bereits von der Recheneinheit erkannte Steckdose in der virtuellen Umgebung 40 bei der Anordnung eines virtuellen Einrichtungselements 50 berücksichtigt werden. Oder in einem anderen Beispiel kann die Recheneinheit 10 erkennen, dass ein Ort bzw. eine virtuelle Position in der virtuellen Umgebung 40 eher ungeeignet für ein virtuelles Einrichtungselement 50 und/oder einen Prozess sein kann, insbesondere wenn der Sensordatensatz 200 auf Störsignale, beispielsweise störende Lichtquellen 34 und/oder störende Temperaturschwankungen an diesem virtuellen Ort hinweist bzw. rückschließen lässt.

Die Recheneinheit 10 kann auch erkennen, dass ein virtueller Ort bereits durch eine Komponente, insbesondere ein virtuelles Einrichtungselement 50 "besetzt" ist und/oder die virtuelle Umgebung 40 nicht genügend Platz zur Anordnung eines speziellen virtuellen Einrichtungselements 50 bietet. In dem Fall kann die Recheneinheit 10 eine andere Option vorschlagen und/oder eine diesbezügliche Fehlermeldung ausgeben.

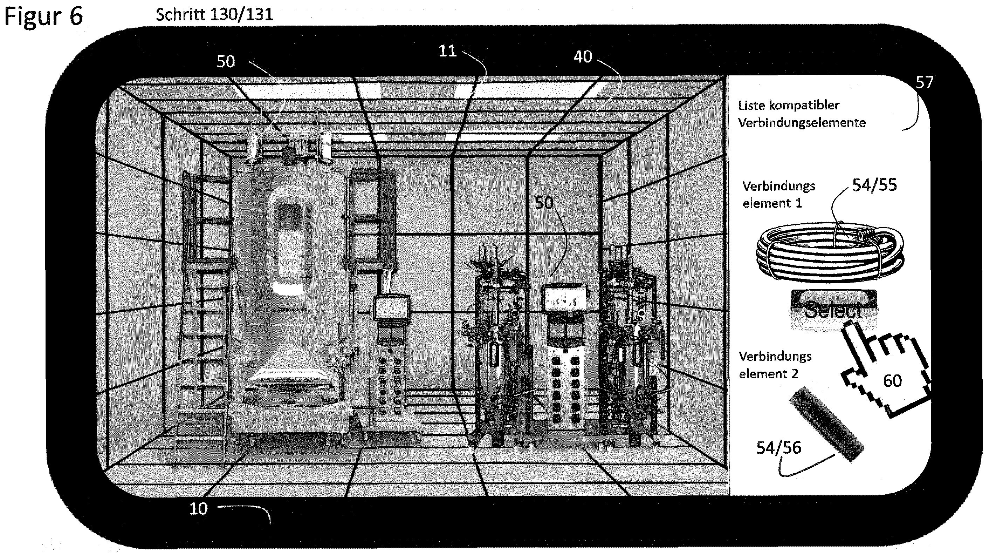

In **Fig. 6** wird ein besonderer Verfahrensschritt 130, insbesondere ein Schritt 131 gemäß einer Ausführungsform näher dargestellt und zwar, der eines Auswählens 131 eines virtuelles Einrichtungselements 50, das einem virtuellen Verbindungselement 54 entspricht. Auf der Benutzeroberfläche bzw. dem Bildschirm 11 wird eine Produktpalette, insbesondere eine Liste 57 kompatibler virtueller Verbindungselemente 54 abgebildet. In der **Fig. 6** sind ein virtueller Schlauch 55 und ein virtuelles Adapter- und/oder Verbindungsstück 56 dargestellt. Ähnlich oder gleich, wie in **Fig. 4** der Schritt des Auswählens 130 bzw. 131 eines virtuellen Einrichtungselements 50 dargestellt ist, kann der Verwender 13 und/oder die Recheneinheit 10 ein virtuelles Verbindungselement 54 aus der angebotenen Auswahl 57 auswählen. Insbesondere handelt es sich auch um eine Vorauswahl von virtuellen Verbindungselementen 54, die einem zuvor ausgewählten virtuellen Einrichtungselement 50 kompatibel sind.

In **Fig. 7** wird ein besonderer Verfahrensschritt 140, insbesondere ein besonderer Schritt 141 gemäß einer Ausführungsform näher dargestellt, und zwar in dem ein virtuelles Verbindungselement 54 in der virtuellen Umgebung 40 angeordnet wird. Besonders bevorzugt umfasst das Verfahren einen Schritt des Erkennens bzw. Identifizierens eines Anschlusses bzw. Konnektors x (Bezugszeichen 59) eines virtuellen Einrichtungselements 50 und/oder eines virtuellen bzw. physischen Anschlusses x, beispielsweise eine Steckdose oder eine Wasserversorgung, der bzw. die zur Infrastruktur der physischen Umgebung 30 gehört. Insbesondere kann das Verfahren einen Schritt des automatischen virtuellen Verbindens von virtuellen Anschlüssen x mittels der virtuellen Verbindungselemente 54 durch der Recheneinheit 10 umfassen. Auch bevorzugt kann das Verfahren ein virtuelles Verbinden virtueller Anschlüsse x durch den Verwender 13 umfassen. Dazu kann die Recheneinheit 10 dem Verwender 13 dadurch assistieren bzw. unterstützen, dass virtuelle Anschlüsse x für den Verwender 13 sichtbar in der virtuellen Umgebung 40 markiert werden. Der Verwender 13 kann durch Anklicken bzw. per "drag-and-drop" das ausgewählte Verbindungselement 54 an den virtuellen Anschlüssen x virtuell anordnen und die virtuellen Einrichtungselemente 50 dadurch virtuell verbinden. Denkbar ist auch, dass der Anschluss x eine AR Markierung zu dessen Erkennung aufweist. Ferner kann der Anschluss x auch selbst als Raummarkierung dienen.

Bevorzugt kann die Recheneinheit 10 in einem Verfahrensschritt unter Einbeziehung der Schwerkraft bzw. Gravitationsrichtung 33 ermitteln, welche Distanz D zwischen zwei virtuellen Anschlüssen x liegt und welche Länge L ein virtuelles Verbindungselement 50 aufweisen muss, damit eine virtuelle Verbindung gelingt. Das virtuelle Verbindungselement 54 ist ein virtuelles Kabel oder ein virtueller Schlauch 55 und "hängt durch" und/oder verläuft zumindest teilweise entlang des virtuellen Bodens B. Insbesondere kann der Verwender 13 die Länge L des virtuellen Verbindungselements 54 manipulieren bzw. ändern. Beispielsweise kann der Verwender 13, eine Länge L in einem Feld der Benutzeroberfläche eingeben und/oder eine Länge L dynamisch mittels eines Sliders verändern. Die Recheneinheit 10 kann in einem bevorzugten Verfahrensschritt auch dazu veranlasst werden, eine Länge L des virtuellen Verbindungselements 54 zu optimieren bezüglich eines Gesichtspunktes.

Im Allgemeinen kann der Schritt des Bestimmens einer virtuellen Umgebung 130, 140 die Schritte 131, 141 und/oder die Schritte 132, 142 umfassen. In anderen Worten können die Schritte 131, 141 unabhängig von den Schritten 132, 142 sein. Bevorzugt umfasst das Bestimmen einer virtuellen Umgebung 130, 140 jedoch die Schritte 132, 142, was den Vorteil hat, dass ein neues virtuelles Einrichtungselement 50 virtuell getestet und möglicherweise von dem Verwender 13 erworben wird.

Die virtuelle Umgebung 40 umfasst insbesondere nach den Schritten 110 bis 140 entsprechend neben den räumlichen Eigenschaften bzw. Merkmalen auch funktionale Merkmale, sofern dem virtuellen Einrichtungselement 50 eine spezielle virtuelle Funktion, beispielsweise ein konkretes Prozessieren eines Mediums, zugeordnet ist.

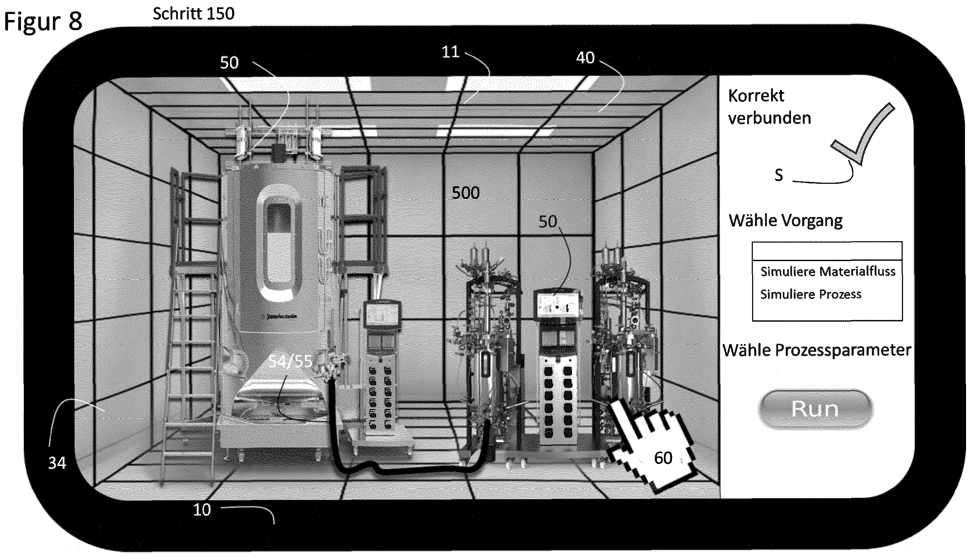

In **Fig. 8** wird der Verfahrensschritt des Vorhersagens 150 (insbesondere des Simulierens) eines Betriebszustandes gemäß einer Ausführungsform näher dargestellt. In einem Verfahrensschritt kann beispielsweise vorhergesagt werden, ob die virtuelle Verbindung 54, 55 korrekt ist. Dies kann bedeuten, dass überprüft wird, ob alle relevanten virtuellen Anschlüsse x miteinander kompatibel sind, die Verbindungselemente 54, 55 die erforderlichen Merkmale, beispielsweise eine geeignete Länge L, aufweisen und die richtigen virtuellen Anschlüsse x gewählt wurden. Die korrekte virtuelle Verbindung kann durch eine entsprechende Statusmeldung S dem Verwender 13 angezeigt bzw. mitgeteilt werden. Eine Fehlermeldung dahingehend würde die fehlerhafte virtuelle Verbindung andeuten. Die Recheneinheit 10 kann (insbesondere zusätzlich) auch auf zumindest ein Problem konkret hinweisen und dem Verwender 13 konkrete Lösungsvorschläge unterbreiten, beispielsweise auf einen geeigneten virtuellen Adapter 56 aus dem Produktsortiment oder auf eine geeignete Länge L des Verbindungselements 54 hinweisen.

Insbesondere in dem Fall, in dem eine Simulation ausgeführt werden soll, kann der Verwender 13 in einem Schritt einen Vorgang aus möglichen Vorgängen auswählen. Beispielsweise kann anhand der verbundenen virtuellen Einrichtungselemente 50, die Behälter aufweisen, ein Materialfluss simuliert bzw. berechnet werden. Dazu kann der Verwender 13 auch Prozessparameter 400 wählen und/oder eingeben und/oder ändern.

Beispielsweise kann der Verwender 13 angeben, wie groß das Volumen des Mediums sein soll und in welcher Zeit das Medium von einem virtuellen Einrichtungselement 50 zu einem anderen transferiert werden soll. Die Recheneinheit führt die gefragte Operation bzw. Berechnung bzw. Simulation mit Hilfe einer Funktion aus. Die statischen oder dynamischen Parameter, die sich aus der Berechnung ergeben, beispielsweise ein Fluss, ein Druck oder ähnliches, können dem Verwender 13, beispielsweise in einem Logfile mitgeteilt bzw. übermittelt werden. Die Simulation kann insbesondere auch visualisiert werden und auf dem Bildschirm 11 "abgespielt" werden. Der Verwender 13 kann insbesondere Parametereinträge von variablen Größen der Parameter- 300 und/oder Sensor- 200 und/oder Prozessparameterdatensätze 400 verändern.

Der Verwender 13 kann beispielsweise auch einen Prozess, beispielsweise einen biochemischen Prozess wählen, sofern dieser mitsamt des benötigten Modells zur Berechnung bzw. Simulation in zumindest einer Datenbank für einen Zugriff hinterlegt ist. Insbesondere kann der Verwender 13 in einem Schritt einen Prozessparameter 400 bestimmen. Beispielsweise kann eine Photosynthese von Algen in einem Medium simuliert werden. In der Simulation können dann beispielsweise unter anderem ein oder mehrere der folgenden Parameter verschiedener Parameterdatensätze berücksichtigt und/oder berechnet werden: Temperatur(schwankung), Lichteinfall, Volumen des Mediums, Menge der Nährstoffe, Lichteinfall, Dauer des Prozesses, Druck und/oder Rührgeschwindigkeit und ähnliche Größen. Einige Parameter können festgehalten werden bzw. invariabel sein, wohingegen andere Parameter flexibel bzw. variabel sind. Insbesondere werden auch thermodynamische relevante Prozesse simuliert.

Beispielsweise kann der Verwender 13 mittels eines "Run-Buttons" die Recheneinheit 10 veranlassen, den gewünschten Vorgang bzw. einen Betriebszustand vorherzusagen und/oder zu simulieren.

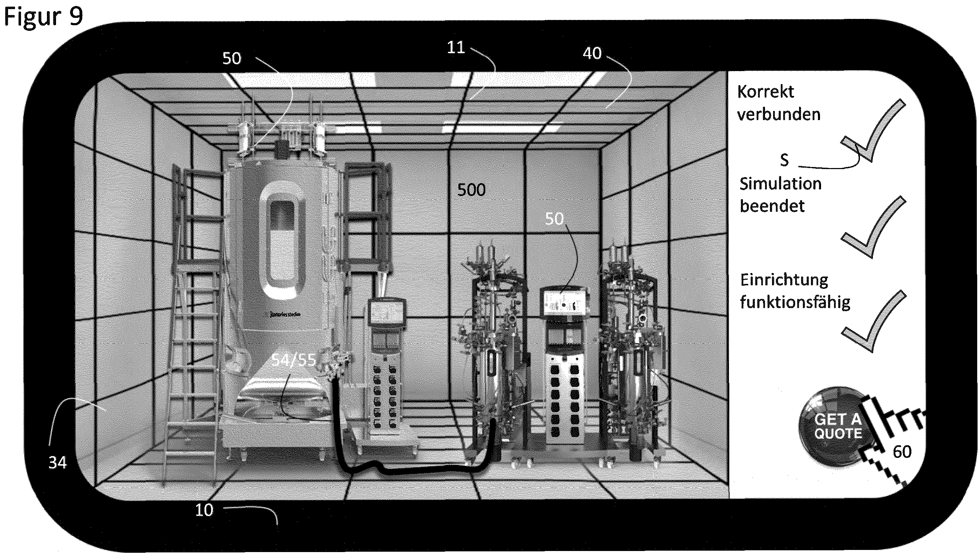

In **Fig. 9** wird der Verfahrensschritt einer Statusmeldung und der Angebotsanfrage 160, 170 gemäß einer Ausführungsform näher dargestellt. Wenn alle Betriebszustände auf Basis der Vorhersage bzw. Simulation als unbedenklich bzw. korrekt bewertet werden, also wenn es insbesondere keine Fehlermeldung gibt, kann dem Verwender 13 erlaubt werden, ein Angebot für die wesentlichen virtuellen bzw. physischen Einrichtungselemente 50 anzufragen. Beispielsweise kann ein "get a quote"-Button bereitgestellt werden, der dazu dient, dass eine Angebotsanfrage an einen oder mehrere Anbieter durch ein Klicken gestellt werden kann.

Die Recheneinheit 10 kann die Angebotsanfrage direkt verarbeiten und/oder weiterleiten. In einem weiteren Schritt 180 kann ein Angebot erstellt werden. Der Verwender 13 kann daraufhin entscheiden, ob er die Ware, insbesondere ein oder mehrere physische Einrichtungselemente 51 der konfigurierten Einrichtung 500 auf Basis des Angebots und/oder der Simulationsresultate bestellen möchte.

**Fig. 10** zeigt eine virtuelle Umgebung 40 gemäß einer Ausführungsform, in der eine virtuelle Einrichtung 500 angeordnet und/oder positioniert ist. In der virtuellen Umgebung 40 ist eine Abbildung 52 eines physischen Einrichtungselements 51 dargestellt, welches bevorzugt mittels des Sensordatensatzes 200 identifiziert und einem virtuellen Einrichtungselement 50 (sowie bevorzugt dessen Parameterdatensatz 300) zugeordnet ist. In anderen Worten kann aufgrund des Sensordatensatzes 200 ein physisches Einrichtungselement 51 erkannt bzw. detektiert bzw. identifiziert und der virtuellen Umgebung 40 als virtuelles Element zugeordnet werden. Ferner ist in der virtuellen Umgebung 40 ein weiteres virtuelles Einrichtungselement 50 in der virtuellen Umgebung 40 angeordnet. Das virtuelle Einrichtungselement 50 ist mittels eines virtuellen Verbindungselements 54 bzw. mittels eines virtuellen Schlauchs 55 virtuell mit dem virtuellen Einrichtungselement 50, das dem physischen Einrichtungselement 51 entspricht, verbunden.

Die Ausführungsform des Verfahrens zur Konfigurierung einer Einrichtung 500 mittels einer erweiterten Realität (augmented reality) der **Fig. 10** umfasst das Anzeigen einer Werkzeugleiste bzw. eines Toolbars T. Die Werkzeugleiste T erlaubt es dem Verwender 13, die Recheneinheit 10 zu veranlassen, eine Schlauchlänge L des virtuellen Schlauchs 55 zu ermitteln ("calculate tube length"). Auf der Werkzeugleiste T sind weitere Elemente einer Benutzeroberfläche BO abgebildet, beispielsweise ein Kontrollfeld zur Änderung und/oder Bestimmung von Parametereinträgen 300 bezüglich des virtuellen Schlauchs 54 ("edit tube"). Ferner kann der Verwender 13 den virtuellen Schlauch 55 virtuell über den virtuellen Boden B mittels dem Werkzeugleistenelement ("set tube to ground") und mittels der dynamischen Krümmungsanpassung ("adapt tube curvature") verlegen. Darüber hinaus kann die erstellte Konfiguration der virtuellen Einrichtung 500 zurückgesetzt ("reset configuration") und/oder gespeichert werden ("save configuration").

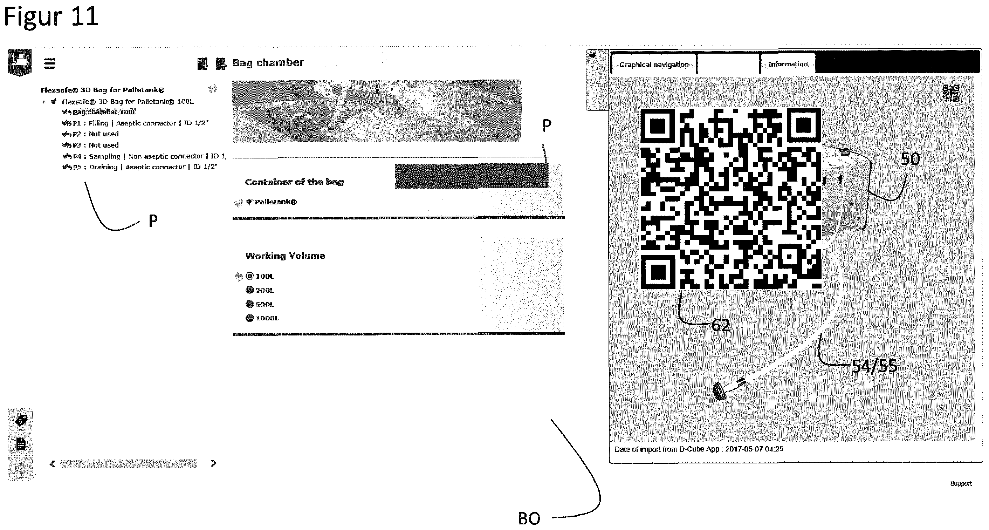

**Fig. 11** zeigt eine Benutzeroberfläche BO gemäß einer Ausführungsform. Die Benutzeroberfläche BO kann durch eine Anwendung erzeugt und auf einen Bildschirm 11 abgebildet werden. In einem Feld wird ein virtuelles Einrichtungselement 50 zur Auswahl abgebildet. Im dargestellten Beispiel handelt es sich dabei um einen virtuellen Kunststoffbehälter, insbesondere um einen virtuellen Kunststoff-Einwegbeutel, der zudem ein virtuelles Verbindungselement 54, nämlich einen virtuellen Schlauch 55 aufweist. Ferner ist eine Identitätsmarkierung 62 in Form einer QR Markierung gemäß einer QR-Kodierung abgebildet. Die Identitätsmarkierung 62 kann beispielsweise von dem Verwender 13 ausgedruckt und an einem Ort in der physischen Umgebung 30 positioniert werden, sodass die Recheneinheit 10 diese Identitätsmarkierung 62 in der virtuellen Umgebung 40 abbilden kann und das entsprechende virtuelle Einrichtungselement 50 virtuell an der entsprechenden virtuellen Stelle anordnen kann.

Es ist fernen ein Parameterfeld P ("Bag Chamber") abgebildet, auf dem ein oder mehrere Felder zur Eingabe und/oder Bestimmung von Parametern, insbesondere eines Parameterdatensatzes 300 bereitgestellt sind. Beispielsweise kann der Verwender 13 einen geeigneten virtuellen Behälter unter einem Feld ("Container of the bag") auswählen durch Anklicken. Es wurde bereits das Produkt bzw. virtuelle Einrichtungselement 50 "Palletank^{®}" gewählt, wie es der angezeigte Haken, sowie das ausgefüllte Feld indiziert. Ferner wurde auch ein Volumen von "100 L" unter dem Feld "Working Volume" aus der abgebildeten Auswahl zwischen 100 L, 200 L, 500 L und 1000 L gewählt. Die Gewählten Parameter sind Bestandteil eines Parameterdatensatzes 300, durch den der gezeigte virtuelle Behälter 50 bestimmt bzw. charakterisiert wird. Der virtuelle Behälter 50 entspricht insbesondere einem Produkt, nämlich einem physischen Einrichtungselement 51, welches erhältlich ist bzw. der Anbieter vertreibt. Beispielsweise kann das Produkt schon existieren, oder aber das Produkt wird nach der Konfiguration für den Verwender 13 erst hergestellt.

Darüber hinaus ist auf der Benutzeroberfläche BO ein weiteres ein Parameterfeld P "Flexsafe^{®} 3D Bag for Palletank^{®}" abgebildet innerhalb dessen, weitere Parameter eines Parameterdatensatzes 300 des virtuellen Beutels namens "Palletank" ausgewählt und/oder bestimmt werden können. Derart kann ein Verwender 13 ein virtuelles Einrichtungselement 50 vor der Anordnung in einer virtuellen Umgebung 40 mit Bezug auf ein oder mehrere mögliche Parameter konfigurieren.

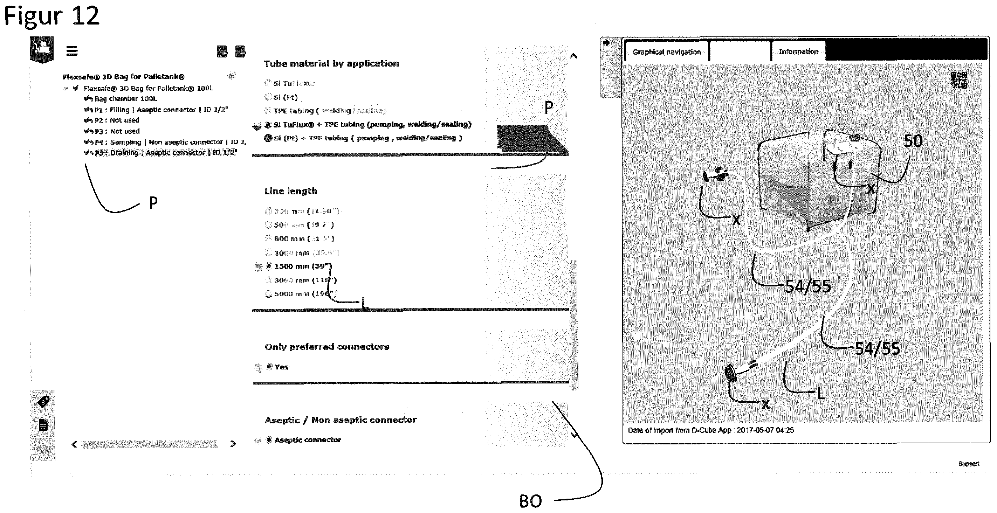

**Fig. 12** zeigt eine Benutzeroberfläche BO gemäß einer Ausführungsform, welche bereits teilweise anhand der **Fig. 11** beschrieben wurde. Es wird der virtuelle Beutel bzw. Behälter 50 mit Anschlüssen x, sowie virtuellen Verbindungselementen 54, nämlich zwei virtuellen Schläuchen 55 mit jeweils virtuellen Anschlüssen x dargestellt.

Mittels des Parameterfeldes P namens "Flexsafe^{®} 3D Bag for Palletank^{®}", kann der Verwender 13 bestimmen, dass er ein virtuelles Verbindungselement 54 namens "Aseptic Connector" konfigurieren kann. In dem damit verbundenen Parameterfeld P namens "Tube material by application" kann der Verwender Parameter, wie Material und Länge L des virtuellen Schlauchs bestimmen. Beispielsweise wurde bereits eine Länge von "1500 mm (59")" aus einer Auswahl zwischen 300 mm, 500 mm, 800 mm, 1000 mm, 1500 mm, 3000 mm und 5000 mm gewählt bzw. bestimmt.

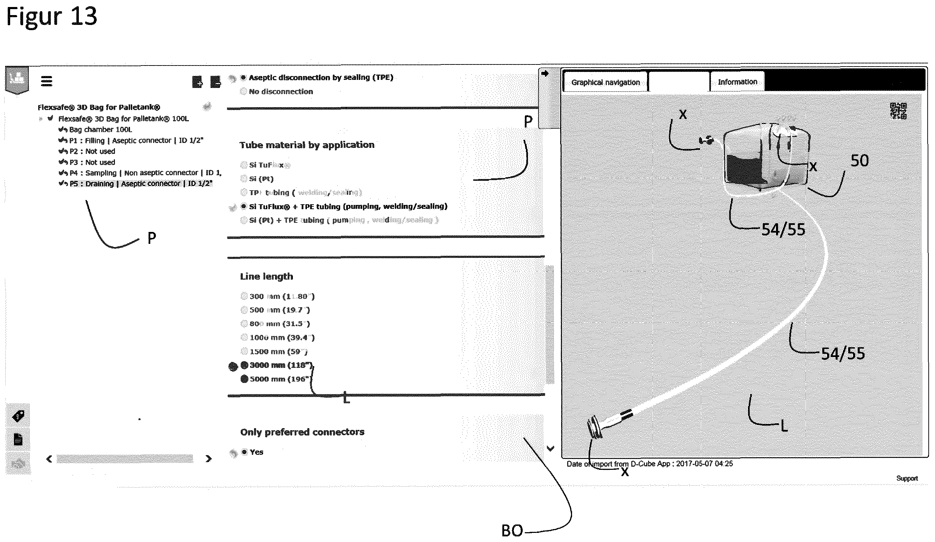

**Fig. 13** zeigt eine Benutzeroberfläche BO gemäß einer Ausführungsform, welche bereits teilweise anhand der **Fig. 11** und **12** beschrieben wurde. Insbesondere wird anhand der **Fig. 13** deutlich, wie die Darstellung des virtuellen Einrichtungselement 50, insbesondere der virtuelle Schlauch 55 durch die Parameterbestimmung durch den Verwender 13 angepasst wird. Der Verwender 13 hat hierin bestimmt, dass der virtuelle Schlauch 55 anstatt "1500 mm (59")" eine Länge L von "3000 mm (59")" haben soll. In der Darstellung des virtuellen Schlauchs 55 der **Fig. 13** kann man erkennen, dass der virtuelle Schlauch entsprechend länger erscheint als in der Darstellung der **Fig. 12****.** Dieser Verfahrensschritt des sofortigen Anpassens visuell dargestellter virtueller Einrichtungselemente 50 hat den Vorteil, dass der Verwender 13 besonders intuitiv bei der Konfiguration einer Einrichtung vorgehen kann.

Allgemein kann ein Verwender 13 des beschriebenen Verfahrens beispielsweise ein Kunde bzw. (potentieller) Einkäufer sein, der Interesse daran hat, sein Labor mit Geräten bzw. einer Einrichtung umfassend ein oder mehrere physische Einrichtungselemente 51 auszustatten, die in einem Online-Katalog bzw. auf einer Webseite zumindest eines Anbieters angeboten werden. Das Labor kann beispielsweise bereits ein oder mehrere Geräte bzw. physische Einrichtungselemente 51 umfassen, die der Anbieter vertreibt oder in der Vergangenheit vertrieben hat. Das Labor kann jedoch auch unbestückt bzw. leer sein und virtuell mit virtuellen Einrichtungselementen 50 bestückt werden.

Eine physische Komponente kann eine reale physische Komponente, beispielsweise ein Möbelstück bzw. eine Laboreinrichtung, insbesondere ein Tisch, ein Stuhl, eine Laborbank, ein Abzug, ein Gestell, ein Regal, eine Ablage und/oder ein physisches Einrichtungselement 51, insbesondere eines, welches durch einen Anbieter vertrieben wird, umfassen. Eine reale physische Komponente kann auch ein Anschluss, insbesondere eine Gas- und/oder Flüssigkeitsabfuhr und/oder -zufuhr, eine Steckdose bzw. ein Netzzugang bzw. -versorgung, ein Lichtschalter, ein Licht, eine Lüftung, ein Fenster, eine Tür und/oder andere Infrastrukturelemente eines Raumes sein bzw. umfassen. Eine Recheneinheit 10 kann dazu ausgelegt sein, ein physisches Einrichtungselement 51 abzubilden und zu identifizieren bzw. erkennen und dieses einem entsprechenden virtuelle Einrichtungselement 50 zuzuordnen, was im Wesentlichen auch einem Transformieren und/oder Digitalisieren eines physischen Einrichtungselements 51 in ein virtuelles Einrichtungselement 50 gleichkommt. Insbesondere kann eine physische reale Komponente also ein registriertes physisches, also reales Einrichtungselement 51 sein. Ein registriertes physisches Einrichtungselement 51 ist ein Einrichtungselement, welches einem Eintrag in einer Tabelle und/oder einem Katalog und insbesondere einem Parameterdatensatz 300 entspricht bzw. zugeordnet ist. Der Eintrag steht bevorzugt für ein physisches Einrichtungselement 51, welches von einem Anwender bestellt und käuflich erworben werden kann und/oder von einem Anbieter zum käuflichen Erwerb angeboten wird und/oder wurde. Eine Komponente kann auch eine virtuelle Komponente, insbesondere ein virtuelles Einrichtungselement 50, beispielsweise ein Einrichtungselement, welches einem Eintrag in einer Tabelle und/oder einem Katalog entspricht, umfassen.

Insbesondere kann das Verfahren zur Konfiguration einer Einrichtung 500 besonders intuitiv sein. Weiterhin kann der Bediener bzw. Benutzer 13 bevorzugt durch Prozessabläufe zur Konfiguration einer Einrichtung 500 geführt werden, ohne dass dieser dafür ein Handbuch benötigt bzw. konsultieren muss.

Die Recheneinheit 10 kann einen Mikrocontroller, einen Mikroprozessor und/oder eine integrierte Schaltung umfassen, die so konfiguriert ist, dass sie Daten von der mindestens einen Sensoreinheit 20 empfängt und Daten an eine Ausgabeeinrichtung, insbesondere einen Bildschirm 11 überträgt. Die Recheneinheit 10 kann Teil eines Computersystems wie eines PCs, eines Smartphones 12 und/oder eines Tablets 12 sein. Insbesondere kann die Recheneinheit 10 eine Recheneinheit eines Gerätes des Verwenders 13 und die Recheneinheit eines entfernten (remote) Gerätes, beispielsweise eines Anbieters von Waren umfassen. In anderen Worten kann die Rechenleistung mehrerer Geräte bzw. Einheiten dazu verwendet werden, eine Rechnung auszuführen. Der Begriff der Recheneinheit 10 kann in dem Fall die einzelnen Rechenuntereinheiten umfassen.

Alle genannten virtuellen Elemente 50 sind jeweils beliebig vereinfachte bzw. beliebig komplexe Modelle für physische reale Elemente 51. Die virtuellen Elemente 50 werden durch Parameter(einträge) eines Parameterdatensatzes 300 bestimmt, die unter anderem anhand der entsprechenden physischen Einrichtungselemente 51 ermittelt bzw. bestimmt wurden und/oder nachträglich variiert wurden. Jedes virtuelle Einrichtungselement 50 kann so gesehen als digitales Modell bzw. digitaler Zwilling des physischen Einrichtungselements 51 dienen. Dies heißt jedoch nicht im Umkehrschluss, dass die Parameter, durch die ein virtuelles Einrichtungselement 50 bestimmt wird auch die Parameter eines physischen Einrichtungselements 51 gänzlich bestimmen. Das virtuelle Einrichtungselement 50 kann dahingehend auch nur als ein vereinfachtes Modell dienen. Die Parameter, durch die ein virtuelles Einrichtungselement 50 und/oder eine andere virtuelle Komponente bestimmt wird, werden in einem Datensatz, beispielsweise einem Parameter- 300 und/oder einem Sensor- 200 und/oder einem Prozessparameterdatensatz 400 geführt.

Der kodierte Informationsgehalt einer QR Markierung gemäß einer QR-Kodierung entspricht im Wesentlichen nicht dem Informationsgehalt einer Augmented Reality AR Markierung. Dennoch kann eine QR Markierung ob ihrer strukturellen Ausgestaltung als eine AR Markierung dienen, nämlich dann wenn lediglich das Muster, nicht aber die Kodierung analysiert wird.

Im Allgemeinen kann eine übliche AR Markierung einer üblichen QR Markierung in der optischen Darstellung auch ähneln, aber in der Regel weisen typische AR Markierungen weniger schwarze und weiße Quadrate auf und sind größer ausgestaltet. Es ist nicht das Ziel einer AR Markierung einen String gemäß einer QR Kodierung zu vermitteln, wohingegen eine QR Markierung einen String übermittelt bzw. einem String entspricht. Ein System, welches auf der AR Markierung basiert, kann die Position und die Orientierung der von einer Kamera identifizierten AR Markierung in 3D erkennen bzw. von der AR Markierung ableiten. In anderen Worten kann eine AR Markierung Informationen umfassen, insbesondere zweidimensionale Muster, die es erlauben, die Position und die Orientierung der AR Markierung im Raum davon abzuleiten, indem die perspektivische Darstellung der AR Markierung auf einem Bild analysiert wird.

Die Auswertung einer QR Markierung mittels einer Entschlüsselung einer QR-Kodierung und einer AR Markierung gestalten sich im Wesentlichen sehr unterschiedlich. Aus einer QR Markierung wird ein Wert aus schwarzen und weißen Quadraten gemäß einer Kodierung ausgelesen, es wird in der Regel in diesem Schritt jedoch keine Position und keine Orientierung ausgelesen. Eine AR-Markierung wird hingegen aus einer Gruppe bekannter AR-Markierungen identifiziert und gleichzeitig wird deren Position und Orientierung in Echtzeit analysiert. Insbesondere können derart auch Bewegungen eines Objektes mit einer AR Markierung versehen analysiert werden.

AR Markierungen sind typischerweise etwas größer als QR Markierungen und können mittels Augmented Reality-Systemen leicht verfolgt werden. AR Markierungen müssen nicht zwingend ausgelesen oder entschlüsseln werden. Eine AR Markierung kann auch lediglich erkannt bzw. identifiziert werden und eine Position und/oder Orientierung kann von der Lage der AR Markierung im Raum abgeleitet werden. Insbesondere kann eine Bewegung der AR Markierung verfolgt werden. Darauf basierend kann eine interaktive 3D-Animation und/oder eine virtuelle Umgebung 40 mit einem virtuellen Einrichtungselement 50 entsprechend und in Echtzeit wiedergegeben werden. Insbesondere ist ein Augmented Reality-System dazu ausgelegt, ohne zusätzliche Markierungen auszukommen. Das bedeutet, dass es in dem Fall nicht nötig ist, dass ein Verwender eine Markierung anbringen bzw. bereitstellen muss, was als "Markerless Tracking" bezeichnet wird. Dies bedeutet dennoch nicht, dass es überhaupt keine Markierung gibt. Stattdessen können bereits bestehende Merkmale in einem Bild als Markierungen dienen. Beispielsweise könnte das Logo eines Unternehmens oder ein Bild auf einem Objekt als AR Markierung dienen, welche ohne weitere Handlung des Verwenders ohnehin in der physischen Umgebung 30, sowie auf der Abbildung der physischen Umgebung 31 präsent wären. Dies könnte eine zusätzliche Anbringung einer (schwarzweiß) AR Markierung durch den Verwender überflüssig machen.

### Bezugszeichen

- 10: Recheneinheit
- 11: Bildschirm
- 12: Smart Device und/oder Smartphone und/oder Tablet
- 13: Verwender
- 20: Sensoreinheit
- 21: Kamera
- 30: physische Umgebung
- 31: Bild bzw. Abbildung der physischen Umgebung
- 32: Raster und/oder digitalisierte 3D-Koordinaten
- 33: Raummarkierung, insbesondere Gravitationsrichtung bzw. Markierung zur Angabe der Gravitationsrichtung
- 34: Reale Lichtquelle
- 35: Virtuelle Lichtquelle
- 40: virtuelle Umgebung
- 50: virtuelles Einrichtungselement
- 51: physisches Einrichtungselement
- 52: Abbildung eines physischen Einrichtungselements
- 54: virtuelles Verbindungselement
- 55: virtueller Schlauch
- 56: virtuelles Adapterelement
- 57: Vorgeschlagene und/oder Präsentierte Auswahl virtueller Einrichtungselemente und/oder Katalogelemente und/oder Produktliste und/oder -auswahl
- 58: Ende eines Verbindungselementes
- 60: Mauszeiger
- 61: Objektmarkierung kann AR Markierung und/oder QR Markierung sein
- 62: Identitätsmarkierung kann AR Markierung und/oder QR Markierung sein
- 100: Schritt des Bereitstellens eines Augmented Reality-Systems
- 110: Schritt des Aufnehmens eines Sensordatensatzes
- 120: Schritt des Bestimmens einer virtuellen Umgebung
- 130: Schritt des Bestimmens einer virtuellen Anordnung - Auswählen
- 131: Bereitstellen einer Produktauswahl und/oder einer Liste von virtuellen Einrichtungselementen und Auswählen eines virtuellen Einrichtungselements
- 132: Bereitstellen, Identifizieren und Zuordnen eines physischen Einrichtungselements
- 140: Schritt des Bestimmens einer virtuellen Anordnung - Anordnen
- 141: Automatisches und/oder manuelles Anordnen eines virtuellen Einrichtungselements
- 142: Automatisches Anordnen
- 150: Schritt des Vorhersagens bzw. des Treffens einer Vorhersage, insbesondere Simulieren einer Funktion und/oder eines Betriebszustandes
- 160: Erlauben einer Angebotsanfrage und/oder Warenbestellungsanfrage
- 170: Erstellen eines Angebots
- 180: Erstellen eines Angebots
- 200: Sensordatensatz
- 300: Parameterdatensatz
- 400: Prozessparameterdatensatz
- 500: Einrichtung
- 1000: Augmented Reality-System bzw. System zur Erzeugung einer erweiterten Realität
- B: Virtueller Boden in der virtuellen Umgebung
- BO: Benutzeroberfläche
- D: Distanz zwischen virtuellen Anschlüssen
- L: Länge eines Verbindungselements
- P: Parameterfeld
- S: Statusmeldung
- T: Werkzeugleiste und/oder Toolbar
- x: Virtueller Anschluss

## Patentansprüche

1. Verfahren zum virtuellen Konfigurieren einer Einrichtung (500), insbesondere Bioprozesseinrichtung, wobei das Verfahren folgende Schritte umfasst:
- Bereitstellen (100) eines Augmented Reality-Systems (1000) umfassend: eine Recheneinheit (10); zumindest eine Sensoreinheit (20) und einen Bildschirm (11);
- Detektieren (110) eines einer physischen Umgebung (30) zugehörigen Sensordatensatzes (200) mittels der zumindest einen Sensoreinheit (20);
- Bestimmen (120) einer virtuellen Umgebung (40) basierend auf dem Sensordatensatz (200) mittels der Recheneinheit (10);
- Bestimmen (130, 140) einer virtuellen Anordnung von zumindest einem virtuellen Einrichtungselement (50) bezüglich der virtuellen Umgebung (40) mittels der Recheneinheit (10), wobei dem virtuellen Einrichtungselement (50) jeweils ein Parameterdatensatz (300) zugeordnet ist;
- Abbilden der virtuellen Umgebung (40) und des zumindest einen virtuellen Einrichtungselementes (50) in Form einer Augmented Reality-Abbildung der virtuellen Anordnung auf dem Bildschirm (11); und
- Erzeugen einer Vorhersage (150) einer Funktionalität und/oder eines Betriebszustandes von zumindest einem Teil der Einrichtung (500) basierend auf dem Parameterdatensatz (300) und dem Sensordatensatz (200) mittels der Recheneinheit (10),
wobei die zumindest eine Sensoreinheit (20) eine Kamera (21) und zumindest einen Gravitationssensor umfasst,
wobei der Schritt des Detektierens (110) des der physischen Umgebung (30) zugehörigen Sensordatensatzes (200) ferner einen Schritt eines Aufnehmens mittels der Kamera (21) eines Bildes (31) der physischen Umgebung (30) umfassend eine räumliche Information (32) umfasst,
wobei das zumindest eine virtuelle Einrichtungselement (50) zumindest ein virtuelles Verbindungselement (54) umfasst,
wobei das Bestimmen (140) der virtuellen Anordnung ein Bestimmen von mindestens drei virtuellen Einrichtungselementen (50) umfasst, wobei eines der mindestens drei virtuellen Einrichtungselemente (50) dem virtuellen Verbindungselement (54) entspricht; wobei der Parameterdatensatz (300), der dem virtuellen Verbindungselement (54) zugeordnet ist, eine Länge (L) des virtuellen Verbindungselementes (54) umfasst;
wobei zwei andere der mindestens drei virtuellen Einrichtungselemente (50) jeweils mindestens einen Anschluss (59) zum Anschließen jeweils eines Endes (58) des virtuellen Verbindungselements (54) umfassen; und
wobei das virtuelle Verbindungselement (54) ein virtuelles Kabel oder ein virtueller Schlauch ist, welches oder welcher zumindest teilweise entlang eines virtuellen Bodens (B) der virtuellen Umgebung (40) verläuft; und das Verfahren ferner folgende Schritte umfasst:
- Bestimmen einer virtuellen Distanz (D) zwischen den mindestens zwei Anschlüssen (59) in der virtuellen Umgebung (40) basierend auf dem Sensordatensatz (200), dem Parameterdatensatz (300) und der virtuellen Anordnung, mittels der Recheneinheit (10);
- Detektieren einer Gravitationsrichtung (33) anhand des zumindest einen Gravitationssensors, der in der physischen Umgebung (30) angeordnet ist oder in einem Detektor eines Smart Device oder Smartphone oder Tablet enthalten ist;
- Bestimmen der virtuellen Länge (L) des Verbindungselementes (54) basierend auf der Distanz (D) zwischen den mindestens zwei Anschlüssen (59) in der virtuellen Umgebung (40); wobei bei der Bestimmung der virtuellen Länge (L) eine Anordnung des Verbindungselementes (54) in der virtuellen Umgebung (40) unter Berücksichtigung der detektierten Gravitationsrichtung erfolgt; und wobei auf dem Bildschirm (11) eine Benutzeroberfläche (BO) abgebildet ist, wobei Elemente der Benutzeroberfläche (BO) auf einer Werkzeugleiste (T) abgebildet sind, die es einem Verwender (13) erlaubt, das virtuelle Verbindungselement (54) über den virtuellen Boden (B) mittels eines Werkzeugleistenelements und einer dynamischen Krümmungsanpassung zu verlegen und die Recheneinheit (10) zum Bestimmen der virtuellen Länge (L) des Verbindungselementes (54) zu veranlassen.

2. Das Verfahren nach Anspruch 1, wobei die zumindest eine Sensoreinheit (20) eine 3D-fähige Kamera umfasst, und der Schritt des Detektierens (110) des der physischen Umgebung (30) zugehörigen Sensordatensatzes (200) ferner einen Schritt eines Aufnehmens einer 3D-Topographie eines Raumes umfasst.

3. Das Verfahren nach Anspruch 1 oder 2, ferner umfassend einen Schritt eines Bereitstellens wenigstens einer Raummarkierung, insbesondere umfassend eine erste Augmented Reality Markierung, in der physischen Umgebung (30), wobei die räumliche Information (32), insbesondere eine 3D-Topologie der physischen Umgebung (30), mittels der Raummarkierung erfasst bzw. spezifiziert werden kann.

4. Das Verfahren nach einem der vorangehenden Ansprüche umfassend den Schritt eines Bereitstellens einer Objektmarkierung (61), insbesondere umfassend eine zweite Augmented Reality Markierung und/oder eine QR Markierung, in der physischen Umgebung (30), wobei ein virtueller Bestimmungsort und/oder eine virtuelle Orientierung bezüglich der virtuellen Umgebung (30) des virtuellen Einrichtungselements und/oder eine Identität des virtuellen Einrichtungselements mittels der Objektmarkierung identifiziert und/oder bestimmt und/oder spezifiziert ist.

5. Das Verfahren nach einem der vorangehenden Ansprüche, ferner umfassend folgende Schritte:
- Erlauben eines Auswählens durch den Verwender (13) und/oder die Recheneinheit (10) zumindest eines virtuellen Einrichtungselements (50), vorzugsweise aus einer Auswahl (57) mehrerer virtueller Einrichtungselemente (50); und insbesondere
- Bereitstellen (132) eines registrierten physischen Einrichtungselements (51) in der physischen Umgebung (30), wobei das Auswählen des zumindest einen virtuellen Einrichtungselements (50) bevorzugt mittels der Recheneinheit (10), insbesondere automatisch erfolgt und wobei das Auswählen des zumindest einen virtuellen Einrichtungselements (50) auf einem Schritt eines Identifizierens des registrierten physischen Einrichtungselements (51) mit einem virtuellen Einrichtungselement (50) insbesondere aus der Auswahl (57) der mehreren virtuellen Einrichtungselemente (50) basiert, wobei bevorzugt der Schritt des Identifizierens des registrierten physischen Einrichtungselements (51) einen Schritt eines Erkennens eines Formmerkmals des physischen Einrichtungselements (51) und/oder einer Identitätsmarkierung (62), insbesondere eine dritte AR Markierung des registrierten physischen Einrichtungselements (51) umfasst.

6. Das Verfahren nach einem der vorangehenden Ansprüche, wobei das zumindest eine virtuelle Einrichtungselement (50) zumindest eines der folgenden umfasst: einen Bioreaktor, einen Einwegbeutel, ein Behältnis, einen Tank, ein Filtersystem, einen Mischeinrichtung, einen Gärtank, eine Zentrifuge, eine Chromatographiesäule, einen Membran-Adsorber, eine Abfülleinrichtung; und insbesondere der Parameterdatensatz (300) zumindest eine variable und/oder eine fixe Größe aus den folgenden umfasst: eine Identifizierungskodierung, eine Bestellnummer, ein Volumen, eine Länge, eine räumliche Ausdehnung, einen Durchmesser, eine Struktur, ein Material, einen Betriebsbereich, einen Betriebsgrenzwert, eine Kompatibilität mit einem weiteren virtuellen Einrichtungselement (50), eine Kompatibilität mit einer biologischen und/oder chemischen Reaktion, einen Parameterdatensatz zu einem Medium und bevorzugt der biologischen und/oder chemischen Reaktion des Mediums.

7. Das Verfahren nach Anspruch 6, wobei das Bestimmen (140) der virtuellen Anordnung ein Bestimmen von mindestens zwei virtuellen Einrichtungselementen (50) aufweist, und das Verfahren ferner folgende Schritte umfasst:
- Prüfen der Kompatibilität zwischen den mindestens zwei virtuellen Einrichtungselementen (50);
- wobei falls die Kompatibilität in der Prüfung festgestellt wurde:
∘ Erlauben eines virtuellen Verbindens der mindestens zwei virtuellen Einrichtungselemente (50) mittels des virtuellen Verbindungselementes (54) durch den Verwender (13) und/oder die Recheneinheit (10);
- wobei falls die Kompatibilität in der Prüfung nicht festgestellt wurde:
∘ Ausgabe einer Fehlermeldung, die das Fehlen der Kompatibilität meldet.

8. Das Verfahren nach einem der vorangehenden Ansprüche, wobei der Bildschirm (11) ein touch screen ist.

9. Das Verfahren nach Anspruch 1, wobei das Bestimmen (140) der virtuellen Anordnung ferner mindestens einen Schritt eines Anordnens per Drag-and-Drop des zumindest einen virtuellen Einrichtungselements (50) mittels des Verwenders (13) und des Bildschirms (11) umfasst.

10. Das Verfahren nach einem der vorangehenden Ansprüche, wobei mindestens eine variable Größe des Parameterdatensatzes (300), insbesondere die Länge (L) des virtuellen Einrichtungselements (50) durch den Verwender (13) am Bildschirm (11), insbesondere durch Betätigung auf dem touch screen, bestimmt werden kann, und/oder wobei der der physischen Umgebung (30) zugehörige Sensordatensatz (200) mindestens eine der folgenden Größen umfasst: eine Temperatur, eine Zeit, eine elektrisches Feldstärke, eine Lichtintensität, eine Vibration, ein Geräusch.

11. Das Verfahren nach einem der vorangehenden Ansprüche, wobei der Schritt des Vorhersagens (150) des Betriebszustandes von dem zumindest einem Teil der Einrichtung (500) einen Schritt eines Simulierens zumindest einer dynamischen Größe eines dynamischen Prozesses umfasst, und zwar basierend auf dem Sensordatensatz (200), dem Parameterdatensatz (300) und der virtuellen Anordnung, wobei der dynamische Prozess zumindest einen aus den folgenden umfasst: ein Materialfluss, ein biologischer Prozess, ein chemischer Prozess, ein physischer Prozess, ein mechanischer Prozess, wobei bevorzugt der Schritt des Vorhersagens (150) eines Betriebszustandes einen Schritt eines Melden eines korrekten Betriebszustandes oder das Melden eines fehlerhaften Betriebszustandes umfasst, insbesondere basierend auf dem Schritt des Simulierens der dynamischen Größe.

12. Das Verfahren nach einem der vorangehenden Ansprüche, ferner umfassend folgende Schritte:
- Erlauben (160) einer Angebotsanfrage für das zumindest eine virtuelle Einrichtungselement (50) basierend auf dem Parameterdatensatz (300);
- Erstellen eines Angebots (180) basierend auf der Angebotsanfrage;
- Erlauben (160) einer Warenbestellungsanfrage;
- Verarbeitung (180) einer Warenbestellung basierend auf der Warenbestellungsanfrage.

13. Das Verfahren nach einem der vorangehenden Ansprüche, umfassend den Schritt einer manuellen Markierung in der virtuellen Umgebung (40) unter Zuhilfenahme eines Bildschirms (11), insbesondere zur Markierung eines virtuellen Raumpunktes und/oder eines virtuellen (50) und/oder physischen Einrichtungselements (51).

14. Computerprogrammprodukt für eine computergestützte virtuelle Konfiguration einer Einrichtung (500), insbesondere einer Bioprozesseinrichtung, umfassend Befehle, die wenn das Computerprogramm von einem Augmented Reality System (1000), umfassend zumindest eine Recheneinheit (10), eine Sensoreinheit (20) und einen Bildschirm (11), ausgeführt wird, das Augmented Reality System veranlassen, das Verfahren nach Anspruch 1 auszuführen.

15. Augmented Reality-System (1000) für eine virtuelle Konfiguration einer Einrichtung (500), insbesondere einer Bioprozesseinrichtung, wobei das Augmented Reality-System (1000) umfasst:
- mindestens eine Sensoreinheit (20), die dazu ausgelegt ist, einen einer physischen Umgebung (30) zugehörigen Sensordatensatz (200) zu erfassen, wobei die Sensoreinheit (20) zumindest einen Gravitationssensor umfasst;
- einen Bildschirm; und
- eine Recheneinheit (10), die dazu ausgelegt ist folgende Schritte auszuführen:
- Bestimmen (120) einer virtuellen Umgebung (40) basierend auf einem einer physischen Umgebung (30) zugehörigen Sensordatensatz (200);
- Bestimmen (130, 140) einer virtuellen Anordnung von zumindest einem virtuellen Einrichtungselement (50) bezüglich der virtuellen Umgebung (40), wobei dem virtuellen Einrichtungselement (50) ein Parameterdatensatz (300) zugeordnet ist;
- Abbilden der virtuellen Umgebung (40) und des zumindest einen virtuellen Einrichtungselementes (50) in Form einer Augmented Reality-Abbildung der virtuellen Anordnung auf dem Bildschirm (11); und
- Vorhersagen (150) einer Funktionalität und/oder eines Betriebszustandes von zumindest einem Teil der Einrichtung (500) basierend auf dem Parameterdatensatz (300) und dem Sensordatensatz (200);
- Bestimmen einer virtuellen Distanz (D) zwischen mindestens zwei Anschlüssen (59) in der virtuellen Umgebung (40) basierend auf dem Sensordatensatz (200), dem Parameterdatensatz (300) und der virtuellen Anordnung;
- Detektieren einer Gravitationsrichtung anhand des zumindest einen Gravitationssensors, der in der physischen Umgebung (30) angeordnet ist und/oder in einem Detektor eines Smart Device und/oder Smartphone und/oder Tablet enthalten ist;
- Bestimmen einer virtuellen Länge (L) eines virtuellen Verbindungselementes (54) basierend auf der Distanz (D) zwischen den mindestens zwei Anschlüssen (59) in der virtuellen Umgebung (40); wobei das virtuelle Verbindungselement (54) ein virtuelles Kabel oder ein virtueller Schlauch ist, welches oder welcher zumindest teilweise entlang eines virtuellen Bodens (B) der virtuellen Umgebung verläuft, wobei bei der Bestimmung der virtuellen Länge (L) eine Anordnung des Verbindungselementes (54) in der virtuellen Umgebung (40) unter Berücksichtigung der detektierten Gravitationsrichtung erfolgt; und
wobei auf dem Bildschirm (11) eine Benutzeroberfläche (BO) abgebildet ist, wobei Elemente der Benutzeroberfläche (BO) auf einer Werkzeugleiste (T) abgebildet sind, die es einem Verwender (13) erlaubt, das virtuelle Verbindungselement (54) über den virtuellen Boden (B) mittels eines Werkzeugleistenelements und einer dynamischen Krümmungsanpassung zu verlegen und die Recheneinheit (10) zum Bestimmen der virtuellen Länge (L) des Verbindungselementes (54) zu veranlassen.

## Claims

1. Method for virtually configuring a piece of equipment (500), in particular bioprocessing equipment, the method comprising the following steps:
- providing (100) an augmented reality system (1000) comprising: a computer unit (10); at least one sensor unit (20) and a screen (11);
- detecting (110) a sensor dataset (200) associated with a physical environment (30) by means of the at least one sensor unit (20);
- determining (120) a virtual environment (40) based on the sensor dataset (200) by means of the computer unit (10);
- determining (130, 140) a virtual arrangement of at least one virtual equipment element (50) relative to the virtual environment (40) by means of the computer unit (10), wherein the virtual equipment element (50) is assigned a respective parameter dataset (300);
- displaying the virtual environment (40) and the at least one virtual equipment element (50) in the form of an augmented reality image of the virtual arrangement on the screen (11); and
- generating a prediction (150) of a functionality and/or an operating status of at least one part of the equipment (500) based on the parameter dataset (300) and the sensor dataset (200) by means of the computer unit (10),
wherein the at least one sensor unit (20) comprises a camera (21) and at least one gravitational sensor,
wherein the step of detecting (110) the sensor dataset (200) associated with the physical environment (30) further comprises a step of recording, by means of the camera (21), an image (31) of the physical environment (30) comprising spatial information (32),
wherein the at least one virtual equipment element (50) comprises at least one virtual connection element (54),
wherein determining (140) the virtual arrangement comprises determining at least three virtual equipment elements (50), wherein one of the at least three virtual equipment elements (50) corresponds to the virtual connection element (54); wherein the parameter dataset (300) associated with the virtual connection element (54) comprises a length (L) of the virtual connection element (54);
wherein two others of the at least three virtual equipment elements (50) each comprise at least one port (59) for connecting a respective end (58) of the virtual connection element (54); and
wherein the virtual connection element (54) is a virtual cable or a virtual tube, which extends at least partially along a virtual ground (B) of the virtual environment (40); and the method further comprises the following steps:
- determining a virtual distance (D) between the at least two ports (59) in the virtual environment (40) based on the sensor dataset (200), the parameter dataset (300) and the virtual arrangement, by means of the computer unit (10);
- detecting a gravitational direction (33) by means of the at least one gravitational sensor, which is arranged in the physical environment (30) or is contained in a detector of a smart device or smartphone or tablet;
- determining the virtual length (L) of the connection element (54) based on the distance (D) between the at least two ports (59) in the virtual environment (40); wherein, when the virtual length (L) is being determined, the connection element (54) is arranged in the virtual environment (40) taking into consideration the detected gravitational direction; and wherein a user interface (BO) is displayed on the screen (11), wherein elements of the user interface (BO) are displayed on a toolbar (T), which makes it possible for a user (13) to move the virtual connection element (54) over the virtual ground (B) by means of a toolbar element and by means of a dynamic curvature adaptation and to cause the computer unit (10) to determine the virtual length (L) of the connection element (54).

2. Method according to claim 1, wherein the at least one sensor unit (20) comprises a 3D-capable camera, and the step of detecting (110) the sensor dataset (200) associated with the physical environment (30) further comprises a step of recording a 3D topography of a space.

3. Method according to claim 1 or 2, further comprising a step of providing at least one spatial marking, in particular comprising a first augmented reality marking, in the physical environment (30), wherein the spatial information (32), in particular a 3D topology of the physical environment (30), can be detected or specified by means of the spatial marking.

4. Method according to any one of the preceding claims, comprising the step of providing an object marking (61), in particular comprising a second augmented reality marking and/or a QR marking, in the physical environment (30), wherein a virtual destination and/or a virtual orientation with respect to the virtual environment (30) of the virtual equipment element and/or an identity of the virtual equipment element is identified and/or determined and/or specified by means of the object marking.

5. Method according to any one of the preceding claims, further comprising the following steps:
- allowing the selection, by the user (13) and/or the computer unit (10), of at least one virtual equipment element (50), preferably from a selection (57) of a plurality of virtual equipment elements (50); and in particular
- providing (132) a registered physical equipment element (51) in the physical environment (30), wherein the selection of the at least one virtual equipment element (50) preferably takes place by means of the computer unit (10), in particular automatically, and wherein the selection of the at least one virtual equipment element (50) is based on a step of identifying the registered physical equipment element (51) using a virtual equipment element (50) in particular from the selection (57) of the plurality of virtual equipment elements (50), wherein preferably the step of identifying the registered physical equipment element (51) comprises a step of recognizing a shape feature of the physical equipment element (51) and/or an identity marking (62), in particular a third AR marking of the registered physical equipment element (51).

6. Method according to any one of the preceding claims, wherein the at least one virtual equipment element (50) comprises at least one of the following: a bioreactor, a disposable bag, a container, a tank, a filter system, a mixing device, a fermentation tank, a centrifuge, a chromatography column, a membrane adsorber, a filling device; and in particular the parameter dataset (300) comprises at least one variable and/or one fixed parameter from the following: an identification coding, an order number, a volume, a length, a spatial extent, a diameter, a structure, a material, an operating range, an operating limit, a compatibility with a further virtual equipment element (50), a compatibility with a biological and/or chemical reaction, a parameter dataset relating to a medium and preferably the biological and/or chemical reaction of the medium.

7. Method according to claim 6, wherein determining (140) the virtual arrangement comprises determining at least two virtual equipment elements (50), and the method further comprises the following steps:
- checking the compatibility between the at least two virtual equipment elements (50);
- wherein if the check finds that they are compatible:
∘ allowing a virtual connection of the at least two virtual equipment elements (50) by means of the virtual connection element (54) by the user (13) and/or the computer unit (10);
- wherein if the check finds that they are not compatible:
∘ outputting an error message reporting the lack of compatibility.

8. Method according to any one of the preceding claims, wherein the screen (11) is a touch screen.

9. Method according to claim 1, wherein determining (140) the virtual arrangement further comprises at least one step of arranging the at least one virtual equipment element (50) by drag-and-drop by means of the user (13) and the screen (11).

10. Method according to any one of the preceding claims, wherein at least one variable parameter of the parameter dataset (300), in particular the length (L) of the virtual equipment element (50), can be determined by the user (13) on the screen (11), in particular by actuation on the touch screen, and/or wherein the sensor dataset (200) associated with the physical environment (30) comprises at least one of the following parameters: a temperature, a time, an electric field strength, a light intensity, a vibration, a noise.

11. Method according to any one of the preceding claims, wherein the step of predicting (150) the operating status of the at least one part of the equipment (500) comprises a step of simulating at least one dynamic parameter of a dynamic process, namely based on the sensor dataset (200), the parameter dataset (300) and the virtual arrangement, wherein the dynamic process comprises at least one of the following: a material flow, a biological process, a chemical process, a physical process, a mechanical process, wherein preferably the step of predicting (150) an operating status comprises a step of reporting a correct operating status or reporting a faulty operating status, in particular based on the step of simulating the dynamic parameter.

12. Method according to any one of the preceding claims, further comprising the following steps:
- allowing (160) a quotation request for the at least one virtual equipment element (50) based on the parameter dataset (300);
- creating a quotation (180) based on the quotation request;
- allowing (160) a goods order request;
- processing (180) a goods order based on the goods order request.

13. Method according to any one of the preceding claims, comprising the step of manually marking in the virtual environment (40) with the aid of a screen (11), in particular for marking a virtual spatial point and/or a virtual (50) and/or physical equipment element (51).

14. Computer program product for virtually configuring a piece of equipment (500), in particular bioprocessing equipment, in a computer-aided manner, the computer program product comprising commands which, when the computer program is executed by an augmented reality system (1000), comprising at least a computer unit (10), a sensor unit (20) and a screen (11), cause the augmented reality system to carry out the method according to claim 1.

15. Augmented reality system (1000) for virtually configuring a piece of equipment (500), in particular bioprocessing equipment, wherein the augmented reality system (1000) comprises:
- at least one sensor unit (20) configured to detect a sensor dataset (200) associated with a physical environment (30), wherein the sensor unit (20) comprises at least one gravitational sensor;
- a screen; and
- a computer unit (10) configured to perform the following steps:
- determining (120) a virtual environment (40) based on a sensor dataset (200) associated with a physical environment (30);
- determining (130, 140) a virtual arrangement of at least one virtual equipment element (50) relative to the virtual environment (40), wherein the virtual equipment element (50) is assigned a respective parameter dataset (300);
- displaying the virtual environment (40) and the at least one virtual equipment element (50) in the form of an augmented reality image of the virtual arrangement on the screen (11); and
- predicting (150) a functionality and/or an operating status of at least one part of the equipment (500) based on the parameter dataset (300) and the sensor dataset (200);
- determining a virtual distance (D) between at least two ports (59) in the virtual environment (40) based on the sensor dataset (200), the parameter dataset (300) and the virtual arrangement;
- detecting a gravitational direction by means of the at least one gravitational sensor, which is arranged in the physical environment (30) and/or is contained in a detector of a smart device and/or smartphone and/or tablet;
- determining a virtual length (L) of a virtual connection element (54) based on the distance (D) between the at least two ports (59) in the virtual environment (40); wherein the virtual connection element (54) is a virtual cable or a virtual tube, which extends at least partially along a virtual ground (B) of the virtual environment, wherein, when the virtual length (L) is being determined, the connection element (54) is arranged in the virtual environment (40) taking into consideration the detected gravitational direction; and
wherein a user interface (BO) is displayed on the screen (11), wherein elements of the user interface (BO) are displayed on a toolbar (T), which makes it possible for a user (13) to move the virtual connection element (54) over the virtual ground (B) by means of a toolbar element and by means of a dynamic curvature adaptation and to cause the computer unit (10) to determine the virtual length (L) of the connection element (54).

## Revendications

1. Procédé pour la configuration virtuelle d'un dispositif (500), en particulier dispositif de bioprocédé, dans lequel le procédé comprend les étapes suivantes :
- la fourniture (100) d'un système de réalité augmentée (1000) comprenant : une unité de calcul (10) ; au moins une unité capteur (20) et un écran (11) ;
- la détection (110) d'un ensemble de données de capteur (200) associé à un environnement physique (30) au moyen de l'au moins une unité capteur (20) ;
- la détermination (120) d'un environnement virtuel (40) sur la base de l'ensemble de données de capteur (200) au moyen de l'unité de calcul (10) ;
- la détermination (130, 140) d'un agencement virtuel d'au moins un élément de dispositif virtuel (50) par rapport à l'environnement virtuel (40) au moyen de l'unité de calcul (10), dans lequel respectivement un ensemble de données de paramètres (300) est associé à l'élément de dispositif virtuel (50) ;
- la représentation de l'environnement virtuel (40) et de l'au moins un élément de dispositif virtuel (50) sous forme d'une représentation en réalité augmentée de l'agencement virtuel sur l'écran (11) ; et
- la génération d'une prédiction (150) d'une fonctionnalité et/ou d'un état de fonctionnement d'au moins une partie du dispositif (500) sur la base de l'ensemble de données de paramètres (300) et de l'ensemble de données de capteur (200) au moyen de l'unité de calcul (10),
dans lequel l'au moins une unité capteur (20) comprend une caméra (21) et au moins un capteur de gravitation,
dans lequel l'étape de la détection (110) de l'ensemble de données de capteur (200) associé à l'environnement physique (30) comprend en outre une étape d'un enregistrement au moyen de la caméra (21) d'une image (31) de l'environnement physique (30) comprenant une information spatiale (32),
dans lequel l'au moins un élément de dispositif virtuel (50) comprend au moins un élément de liaison virtuel (54),
dans lequel la détermination (140) de l'agencement virtuel comprend une détermination d'au moins trois éléments de dispositif virtuels (50), dans lequel l'un des au moins trois éléments de dispositif virtuels (50) correspond à l'élément de liaison virtuel (54) ; dans lequel l'ensemble de données de paramètres (300), qui est associé à l'élément de liaison virtuel (54), comprend une longueur (L) de l'élément de liaison virtuel (54) ;
dans lequel deux autres des au moins trois éléments de dispositif virtuels (50) comprennent respectivement au moins un raccordement (59) pour raccorder respectivement une extrémité (58) de l'élément de liaison virtuel (54) ; et
dans lequel l'élément de liaison virtuel (54) est un câble virtuel ou un tuyau virtuel, lequel s'étend au moins en partie le long d'un sol virtuel (B) de l'environnement virtuel (40) ; et le procédé comprend en outre les étapes suivantes :
- la détermination d'une distance virtuelle (D) entre les au moins deux raccordements (59) dans l'environnement virtuel (40) sur la base de l'ensemble de données de capteur (200), de l'ensemble de données de paramètres (300) et de l'agencement virtuel, au moyen de l'unité de calcul (10) ;
- la détection d'une direction gravitationnelle (33) sur la base de l'au moins un capteur de gravitation, qui est disposé dans l'environnement physique (30) ou est contenu dans un détecteur d'un dispositif intelligent ou téléphone intelligent ou une tablette ;
- la détermination de la longueur virtuelle (L) de l'élément de liaison (54) sur la base de la distance (D) entre les au moins deux raccordements (59) dans l'environnement virtuel (40) ; dans lequel lors de la détermination de la longueur virtuelle (L) un agencement de l'élément de liaison (54) dans l'environnement virtuel (40) s'effectue avec prise en compte de la direction gravitationnelle détectée ; et
dans lequel une interface utilisateur (BO) est représentée sur l'écran (11), dans lequel des éléments de l'interface utilisateur (BO) sont représentés sur une barre d'outils (T), qui permet à un utilisateur (13) de poser l'élément de liaison virtuel (54) sur le sol virtuel (B) au moyen d'un élément de barre d'outils et d'une adaptation de courbure dynamique et pour amener l'unité de calcul (10) à déterminer la longueur virtuelle (L) de l'élément de liaison (54).

2. Procédé selon la revendication 1, dans lequel l'au moins une unité capteur (20) comprend une caméra 3D, et l'étape de la détection (110) de l'ensemble de données de capteur (200) associé à l'environnement physique (30) comprend en outre une étape d'un enregistrement d'une topographie 3D d'un espace.

3. Procédé selon la revendication 1 ou 2, comprenant en outre une étape d'une fourniture d'au moins un marquage de pièce, en particulier comprenant un premier marquage à réalité augmentée, dans l'environnement physique (30), dans lequel l'information spatiale (32), en particulier une topologie 3D de l'environnement physique (30), peut être acquise ou spécifiée au moyen du marquage de pièce.

4. Procédé selon l'une quelconque des revendications précédentes comprenant l'étape d'une fourniture d'un marquage d'objet (61), en particulier comprenant un deuxième marquage à réalité augmentée et/ou un marquage QR, dans l'environnement physique (30), dans lequel une destination virtuelle et/ou une orientation virtuelle par rapport à l'environnement virtuel (30) de l'élément de dispositif virtuel et/ou une identité de l'élément de dispositif virtuel est identifiée et/ou déterminée et/ou spécifiée au moyen du marquage d'objet.

5. Procédé selon l'une quelconque des revendications précédentes, comprenant en outre les étapes suivantes :
- le fait de permettre une sélection par l'utilisateur (13) et/ou l'unité de calcul (10) d'au moins un élément de dispositif virtuel (50), de préférence parmi un choix (57) de plusieurs éléments de dispositif virtuels (50) ; et en particulier
- la fourniture (132) d'un élément de dispositif physique (51) enregistré dans l'environnement physique (30), dans lequel la sélection de l'au moins un élément de dispositif virtuel (50) s'effectue de préférence au moyen de l'unité de calcul (10), en particulier de manière automatique et dans lequel la sélection de l'au moins un élément de dispositif virtuel (50) se base sur une étape d'une identification de l'élément de dispositif physique (51) enregistré avec un élément de dispositif virtuel (50) en particulier à partir du choix (57) des plusieurs éléments de dispositif virtuels (50), dans lequel de préférence l'étape de l'identification de l'élément de dispositif physique (51) enregistré comprend une étape d'une reconnaissance d'une caractéristique de forme de l'élément de dispositif physique (51) et/ou d'un marquage d'identité (62), en particulier un troisième marquage AR de l'élément de dispositif physique (51) enregistré.

6. Procédé selon l'une quelconque des revendications précédentes, dans lequel l'au moins un élément de dispositif virtuel (50) comprend au moins l'un de ce qui suit : un bioréacteur, un sachet à usage unique, un récipient, un réservoir, un système de filtration, un dispositif de mélange, une cuve de fermentation, une centrifugeuse, une colonne chromatographique, un adsorbeur à membrane, un dispositif de remplissage ; et en particulier l'ensemble de données de paramètres (300) comprend au moins une grandeur variable et/ou une grandeur fixe parmi ce qui suit : un code d'identification, un numéro de commande, un volume, une longueur, une étendue spatiale, un diamètre, une structure, un matériau, une zone de fonctionnement, une valeur limite de fonctionnement, une compatibilité avec un autre élément de dispositif virtuel (50), une compatibilité avec une réaction biologique et/ou chimique, un ensemble de données de paramètres concernant un milieu et de préférence la réaction biologique et/ou chimique du milieu.

7. Procédé selon la revendication 6, dans lequel la détermination (140) de l'agencement virtuel présente une détermination d'au moins deux éléments de dispositif virtuels (50), et le procédé comprend en outre les étapes suivantes :
- le contrôle de la compatibilité entre les au moins deux éléments de dispositif virtuels (50) ;
- dans lequel au cas où la compatibilité dans le contrôle aurait été établie :
∘ le fait de permettre une liaison virtuelle des au moins deux éléments de dispositif virtuels (50) au moyen de l'élément de liaison virtuel (54) par l'utilisateur (13) et/ou l'unité de calcul (10) ;
- dans lequel au cas où la compatibilité dans le contrôle n'aurait pas été établie :
∘ l'émission d'un message d'erreur qui signale l'absence de compatibilité.

8. Procédé selon l'une quelconque des revendications précédentes, dans lequel l'écran (11) est un écran tactile.

9. Procédé selon la revendication 1, dans lequel la détermination (140) de l'agencement virtuel comprend en outre au moins une étape d'un agencement par glisser-déposer de l'au moins un élément de dispositif virtuel (50) au moyen de l'utilisateur (13) et de l'écran (11).

10. Procédé selon l'une quelconque des revendications précédentes, dans lequel au moins une grandeur variable de l'ensemble de données de paramètres (300), en particulier la longueur (L) de l'élément de dispositif virtuel (50) peut être déterminée par l'utilisateur (13) sur l'écran (11), en particulier par actionnement sur l'écran tactile, et/ou dans lequel l'ensemble de données de capteur (200) associé à l'environnement physique (30) comprend au moins une des grandeurs suivantes : une température, une heure, une intensité du champ électrique, une intensité lumineuse, une vibration, un bruit.

11. Procédé selon l'une quelconque des revendications précédentes, dans lequel l'étape de la prédiction (150) de l'état de fonctionnement de l'au moins une partie du dispositif (500) comprend une étape d'une simulation d'au moins une grandeur dynamique d'un procédé dynamique, et ce sur la base de l'ensemble de données de capteur (200), de l'ensemble de données de paramètres (300) et de l'agencement virtuel, dans lequel le procédé dynamique comprend au moins un de ce qui suit : un flux de matière, un procédé biologique, un procédé chimique, un procédé physique, un procédé mécanique, dans lequel de préférence l'étape de la prédiction (150) d'un état de fonctionnement comprend une étape d'une signalisation d'un état de fonctionnement correct ou la signalisation d'un état de fonctionnement défectueux, en particulier sur la base de l'étape de la simulation de la grandeur dynamique.

12. Procédé selon l'une quelconque des revendications précédentes, comprenant en outre les étapes suivantes :
- le fait de permettre (160) une demande d'offre pour l'au moins un élément de dispositif virtuel (50) sur la base de l'ensemble de données de paramètres (300) ;
- l'élaboration d'une offre (180) sur la base de la demande d'offre ;
- le fait de permettre (160) une demande de commande de marchandises ;
- le traitement (180) d'une commande de marchandises sur la base de la demande de commande de marchandises.

13. Procédé selon l'une quelconque des revendications précédentes, comprenant l'étape d'un marquage manuel dans l'environnement virtuel (40) à l'aide d'un écran (11), en particulier pour le marquage d'un point spatial virtuel et/ou d'un élément de dispositif virtuel (50) et/ou physique (51).

14. Produit-programme d'ordinateur pour une configuration virtuelle assistée par ordinateur d'un dispositif (500), en particulier d'un dispositif de bioprocédé, comprenant des instructions qui, lorsque le programme informatique est exécuté par un système de réalité augmentée (1000), comprenant au moins une unité de calcul (10), une unité capteur (20) et un écran (11), amènent le système de réalité augmentée à exécuter le procédé selon la revendication 1.

15. Système de réalité augmentée (1000) pour une configuration virtuelle d'un dispositif (500), en particulier d'un dispositif de bioprocédé, dans lequel le système de réalité augmentée (1000) comprend :
- au moins une unité capteur (20) qui est conçue pour acquérir un ensemble de données de capteur (200) associé à l'environnement physique (30), dans lequel l'unité capteur (20) comprend au moins un capteur de gravitation ;
- un écran ; et
- une unité de calcul (10), qui est conçue pour exécuter les étapes suivantes :
- la détermination (120) d'un environnement virtuel (40) sur la base d'un ensemble de données de capteur (200) associé à l'environnement physique (30) ;
- la détermination (130, 140) d'un agencement virtuel d'au moins un élément de dispositif virtuel (50) par rapport à l'environnement virtuel (40), dans lequel un ensemble de données de paramètres (300) est associé à l'élément de dispositif virtuel (50) ;
- la représentation de l'environnement virtuel (40) et de l'au moins un élément de dispositif virtuel (50) sous forme d'une représentation en réalité augmentée de l'agencement virtuel sur l'écran (11) ; et
- la prédiction (150) d'une fonctionnalité et/ou d'un état de fonctionnement d'au moins une partie du dispositif (500) sur la base de l'ensemble de données de paramètres (300) et de l'ensemble de données de capteur (200) ;
- la détermination d'une distance virtuelle (D) entre au moins deux raccordements (59) dans l'environnement virtuel (40) sur la base de l'ensemble de données de capteur (200), de l'ensemble de données de paramètres (300) et de l'agencement virtuel ;
- la détection d'une direction gravitationnelle sur la base de l'au moins un capteur de gravitation, qui est disposé dans l'environnement physique (30) et/ou est contenu dans un détecteur d'un dispositif intelligent et/ou téléphone intelligent et/ou d'une tablette ;
- la détermination d'une longueur virtuelle (L) d'un élément de liaison virtuel (54) sur la base de la distance (D) entre les au moins deux raccordements (59) dans l'environnement virtuel (40) ; dans lequel l'élément de liaison virtuel (54) est un câble virtuel ou un tuyau virtuel, lequel s'étend au moins en partie le long d'un sol virtuel (B) de l'environnement virtuel, dans lequel lors de la détermination de la longueur virtuelle (L) un agencement de l'élément de liaison (54) dans l'environnement virtuel (40) s'effectue avec prise en compte de la direction gravitationnelle détectée ; et dans lequel une interface utilisateur (BO) est représentée sur l'écran (11), dans lequel des éléments de l'interface utilisateur (BO) sont représentés sur une barre d'outils (T), qui permet à un utilisateur (13) de poser l'élément de liaison virtuel (54) sur le sol virtuel (B) au moyen d'un élément de barre d'outils et d'une adaptation de courbure dynamique et d'amener l'unité de calcul (10) à déterminer la longueur virtuelle (L) de l'élément de liaison (54).
